# EUROPEAN PATENT APPLICATION

(11) **EP 4 620 481 A2**
(43) Date of publication of application: **24.09.2025**
(21) Application number: 25192782.8
(22) Date of filing: 05.04.2022
(51) Int. Cl.: A61K 38/16

(54) **RECOMBINANT LIGASE COMPOSITION AND USES THEREOF**

(30) Priority: 05.04.2021 US 202163171031 P; 19.11.2021 US 202163281608 P
(62) Divisional of application: 22719444.6
(71) Applicant: 10X Genomics, Inc., Pleasanton, CA 94588 (US)
(72) Inventor: SHASTRY, Shankar, Pleasanton, CA 94588 (US); CHI, Javelin C., Pleasanton, CA 94588 (US); LUND, Paul Eugene, Pleasanton, CA 94588 (US)
(74) Representative: J A Kemp LLP

(57) **Abstract**

A recombinant ligase composition including a recombinantly-produced ligase isolated from *Acanthocystis turfacea* chlorella virus 1 (ATCV-1) fused to at least one polynucleotide-binding polypeptide is provided. Compositions containing the recombinant ligase composition, vectors encoding the recombinant ligase composition, and methods of making and using the recombinant ligase composition are also provided.

## Description

### CROSS REFERENCE

This application claims priority to and benefit of U.S. Provisional Application No. 63/171,031 titled "Ligases and Uses Thereof" filed on 5 April 2021 and U.S. Provisional Application No. 63/281,608 titled "Recombinant Ligase Composition and Uses Thereof" filed on 19 November 2021, the entire disclosures of which are hereby incorporated by reference for all purposes.

### SEQUENCE LISTING

The instant application contains a Sequence Listing which has been submitted electronically in ASCII format and is hereby incorporated by reference in its entirety. Said ASCII copy, created on April 4, 2022, is named 10416_070WO1_SL.txt and is 23,956 bytes in size.

### FIELD OF DISCLOSURE

The present disclosure relates generally to a recombinant ligase composition including a recombinantly produced ligase isolated *from Acanthocystis turfacea* chlorella virus 1 ("ATCV-1" or "ATCV1" or "ATCV"),further including a recombinantly produced ligase fusion wherein the ligase is fused to at least one polynucleotide-binding polypeptide, compositions containing the recombinant ligase composition, vectors encoding the recombinant ligase composition, and methods of use thereof.

### BACKGROUND

Ligases are enzymes that catalyze the ligation of two molecules by establishing a new chemical bond. DNA ligases are a particular type of ligase that join DNA fragments together. The ability to catalyze the ligation of adjacent single stranded DNA splinted by a complementary RNA strand is a property that is specific to certain DNA ligases, most notably PBCV-1. Efficient ligation of DNA probes hybridized to an RNA template is a key attribute for many applications in the field of nucleic acid analysis and engineering. There is a need for new and effective ligases that can carry out these functions.

### INCORPORATION BY REFERENCE

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawing.
**FIG. 1** shows an aligned amino acid sequence comparison of the ATCV-1 ligase (SEQ ID NO:1) and a PBCV-1 Ligase (SEQ ID NO:6).
**FIG. 2A** and **FIG. 2B** illustrate structural models of the ATCV-1 ligase superimposed on the crystal structure of the PBCV-1 ligase.
**FIG. 3** shows the schematic for an RNA splinted DNA ligation reaction and standard nucleotide sequences used. The signal that is detected is the FAM label on the acceptor DNA molecule. **FIG. 3** discloses SEQ ID NOS: 2-5, respectively, in order of appearance.
**FIG. 4** is an example of a trace from a capillary electrophoresis measurement of RNA templated DNA ligation using ATCV-1 ligase.
**FIG. 5** is a schematic of an RNA splinted DNA ligation. In this scheme, there are two different dyes, one on the acceptor and another on the donor. Having two dyes allows for simultaneous monitoring of the acceptor and donor molecules during the ligation reaction.
**FIG. 6** is an example of RNA splinted DNA ligation monitored with a FAM fluorescent label on the donor DNA and an Atto565 fluorescent label on the acceptor DNA. Unincorporated donor DNA at position 24 is observed in the FAM channel, unincorporated acceptor DNA is observed at position 26 in the Atto565 channel along with Atto565 labeled AppDNA donor signal. The ligated product runs at 50 nucleotides as evidenced by both FAM and Atto565 fluorescent signals.
**FIG. 7** is an example trace of ligation efficiency of ATCV-1 ligase as a function of enzyme concentration.
**FIG. 8** is an example trace of ligation efficiency of ATCV-1 ligase as a function of magnesium concentration.
**FIG. 9** is an example trace of ligation efficiency of ATCV-1 ligase as a function of ATP concentration.
**FIG. 10** is an example trace of ligation efficiency of two different concentrations of ATCV-1 ligase (1µM and 0.1µM) as a function of pH.
**FIG. 11** **A** and **FIG. 11B** are example traces of Cq value (threshold value) from a qPCR measurement of a RASL assay comparing ATCV-1 ligase, PBCV-1 and T4 DNA ligase.

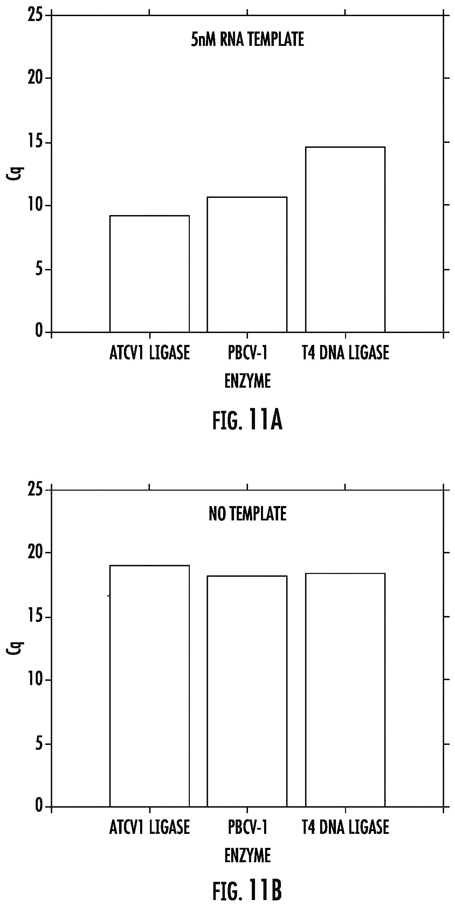

**FIG. 11** **A** illustrates an example trace with 5 nM RNA template; **FIG. 11B** illustrates an example trace with no RNA template.

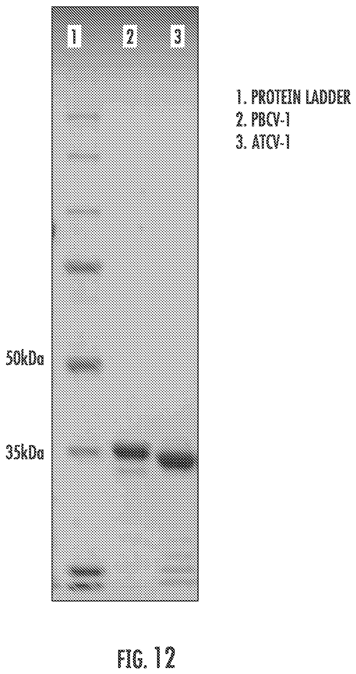

**FIG. 12** is an example of a denaturing SDS PAGE gel showing ATCV-1 ligase alongside PBCV-1. The enzyme runs at approximately 33.71kDa similar to the PBCV-1 enzyme.

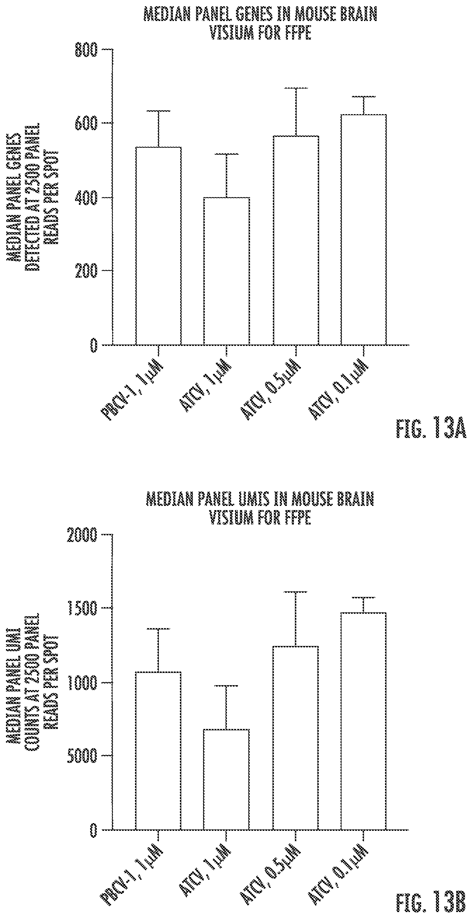

**FIG. 13A** and **FIG. 13B** are graphs illustrating RTL gene expression sensitivity using the Visium for FFPE protocol on mouse brain tissue sections. **FIG. 13A** illustrates a loss of median panel genes downsampled to 2500 median panel reads per spot at 1µM of ATCV-1 ligase compared to PBCV-1 ligase but increased median panel genes at 0.5µM and 0.1µM ATCV-1 ligase; **FIG. 13B** illustrates a loss of median panel UMIs downsampled to 2500 median panel reads per spot at 1µM ATCV-1 ligase compared to PBCV-1 ligase but increased median panel UMIs at 0.5µM and 0.1µM ATCV-1 ligase.

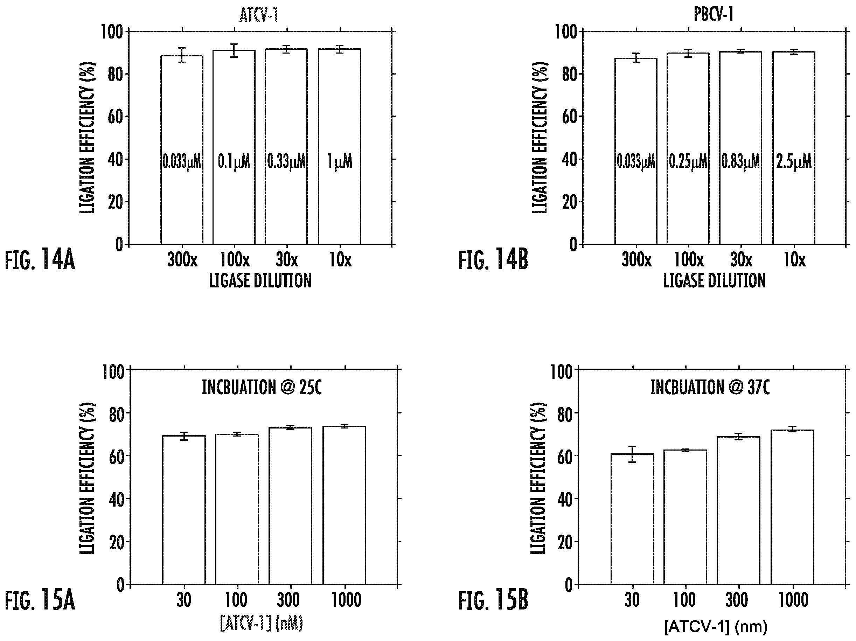

**FIG. 14A** and **FIG. 14B** are examples of a DNA templated DNA ligation using ATCV-1 ligase and PBCV-1, showing that both enzymes function equivalently as DNA ligases under the experimental conditions. **FIG. 14A** illustrates the ATCV-1 ligase activity, while **FIG. 14B** illustrates the PBCV-1 activity.
**FIG. 15A** and **FIG. 15B** are examples of RNA templated DNA ligation by ATCV-1 ligase at two different temperatures, 25°C and 37°C. **FIG. 15A****)** illustrates the RNA templated DNA ligation at 25°C; **FIG. 15B****)** illustrates the RNA templated DNA ligation at 37°C.

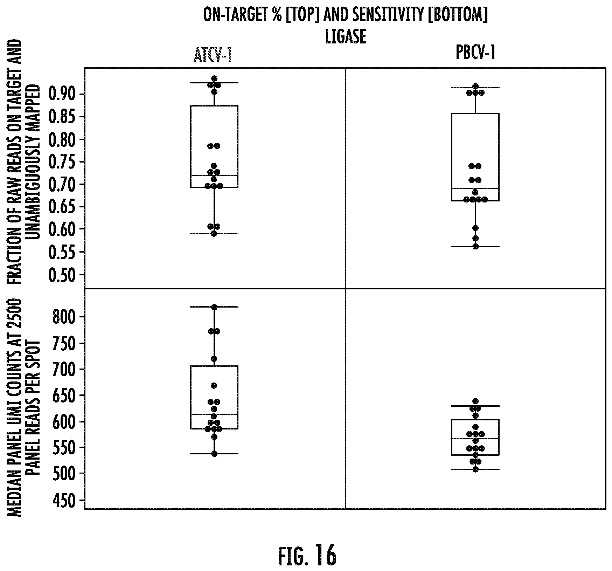

**FIG. 16** provides a graph summarizing single-cell RTL gene expression specificity and sensitivity comparing the performance of ATCV-1 with PBCV-1. Specificity or on target reads (top boxes) refers to the correctly mapped genes, while sensitivity (bottom boxes) refers to the number of UMIs counted, which is a measure of the number of uniquely ligated molecules in a single cell RTL reaction. This data suggests that ATCV-1 performance as measured by specificity and sensitivity is comparable to PBCV-1 performance under the experimental conditions.

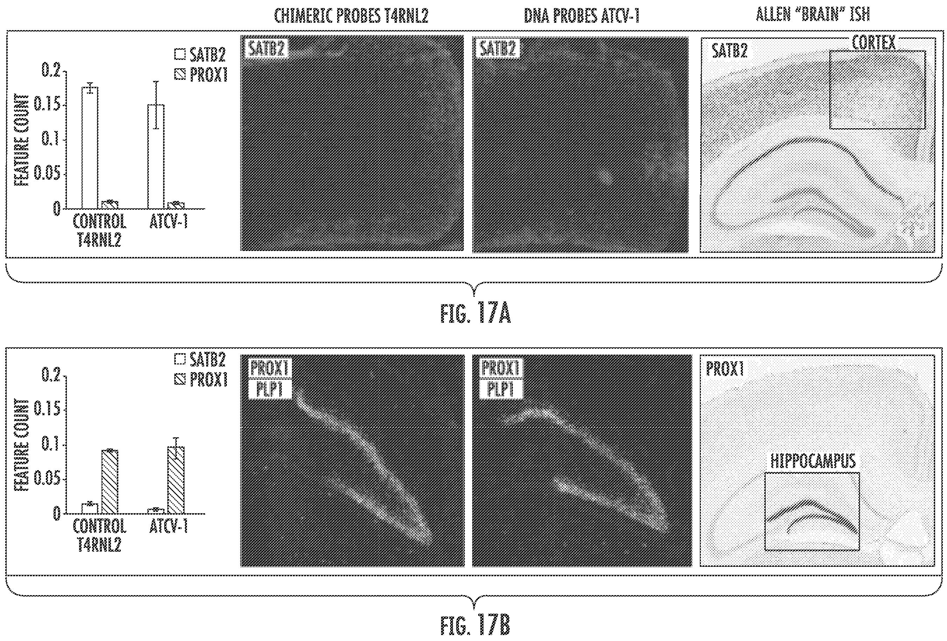

**FIG. 17A** and **FIG. 17B** show that ATCV-1 ligation of DNA padlock probes (middle pictures) performs similar as the positive control using chimeric probes and T4RNA ligase 2 (left pictures), both in terms of sensitivity and the spatial distribution of gene expression in the A) cortex and **B)** hippocampus of a mouse brain. Controls (right pictures) show ISH of the cortex and hippocampal regions in a mouse brain. The graphs demonstrate that the ligation of the probes was specific for the gene targeted (x axis=ligase used, y axis=feature count).

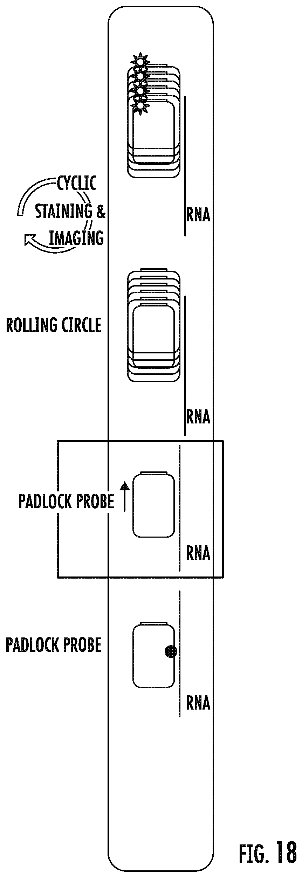

**FIG. 18** is a schematic representation of an *in situ* platform using rolling circle amplification (RCA) where the recombinant ligase composition of the present disclosure could be used.

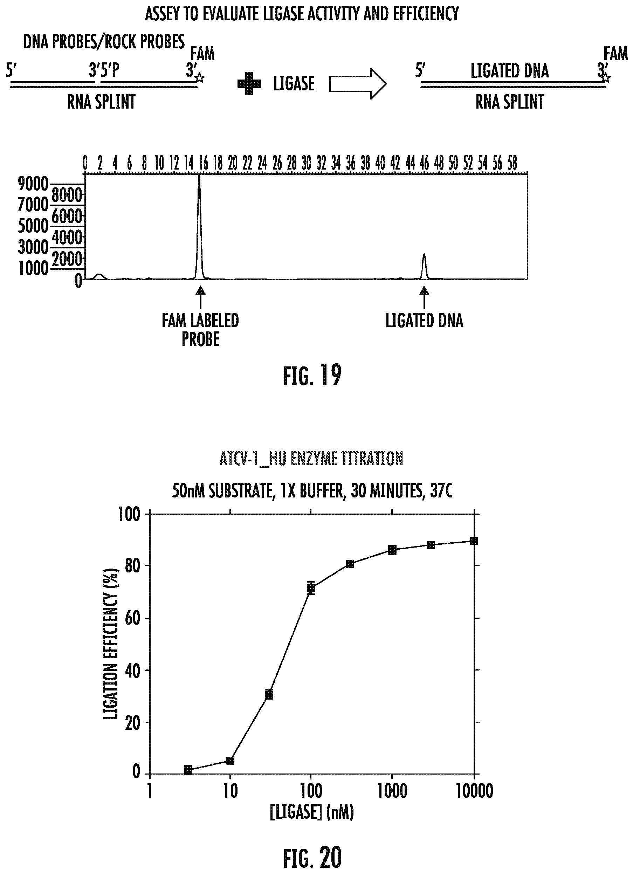

**FIG. 19** is a schematic showing a ligase activity and efficiency assay for evaluating the recombinant ligase composition of the present disclosure.
**FIG. 20** is a graph showing ATCV-1-HU fusion enzyme titration results. Experimental conditions included 50nM of the substrate in 1x buffer with incubation at 37°C for 30 min.

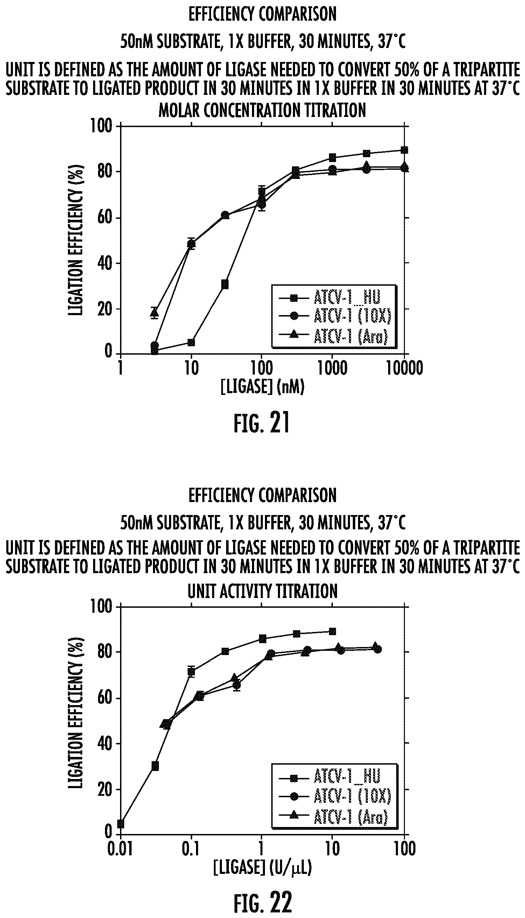

**FIG. 21** is a graph showing efficiency results comparing different molar concentrations of ATCV-1 and ATCV-1-HU fusion enzyme. Experimental conditions were the same as for **FIG. 20****.**
**FIG. 22** is a graph showing efficiency results of a unit activity titration comparing ATCV-1 with ATCV1-HU. One unit is defined as the amount of ligase needed to convert 50% of a tripartite substrate to ligated product under the experimental conditions as found in FIG. 20.

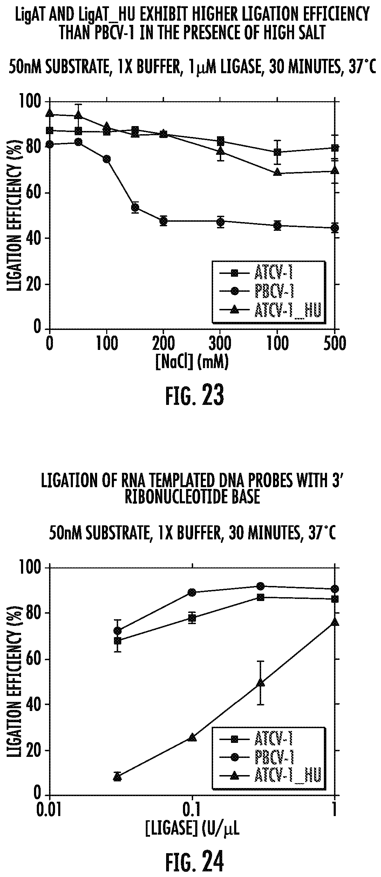

**FIG. 23** **is** a graph showing ligation efficiency results comparing ATCV-1, ATCV-1-HU, and PBCV-1 in the presence of different salt concentrations. Experimental conditions were the same as for **FIG. 20****.**
**FIG. 24** is a graph showing ligation efficiency results for ATCV-1, ATCV-1-HU and T4Rnl2 for ligation of RNA templated DNA probes with a 3' ribonucleotide base. Experimental conditions were the same as for **FIG. 20****.**

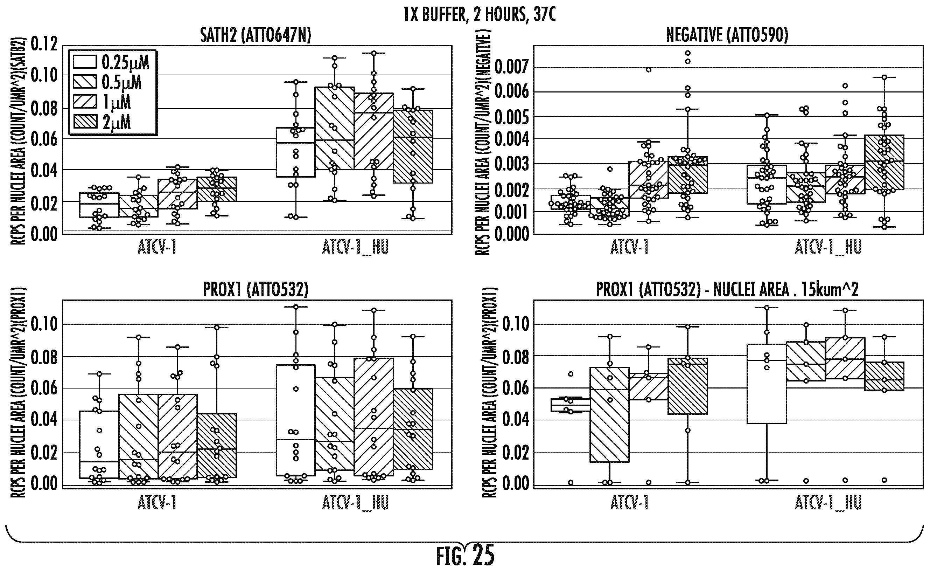

**FIG. 25** are box and whisker charts of *in situ* mouse brain ligation comparisons of ATCV-1 and ATCV-1-HU with different concentrations of HU targeting either Satb2 (top left, with ATTO647N fluorescent moity), Prox1 (bottom left, ATTO532 fluorescent moiety), Prox 1 in the nuclei (bottom right, ATTO532 fluorescent moiety) and Negative control (top right, ATTO590 fluorescent moiety). Regardless of graph; 0.25µM of the ligase (left most box/whisker), 0.5µM of the ligase (second from left box/whisker), 1µM of the ligase (second from right box/whisker), and 2µM of the ligase (right most box/whisker). X axis=ATCV-1 or ATCV-1-HU; Y axis=rolling circle products/nuclei area (counts/um^2).

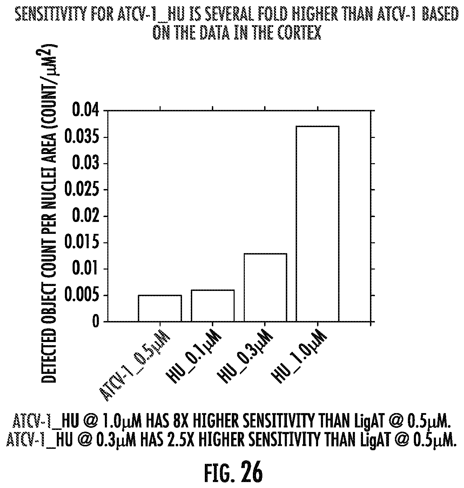

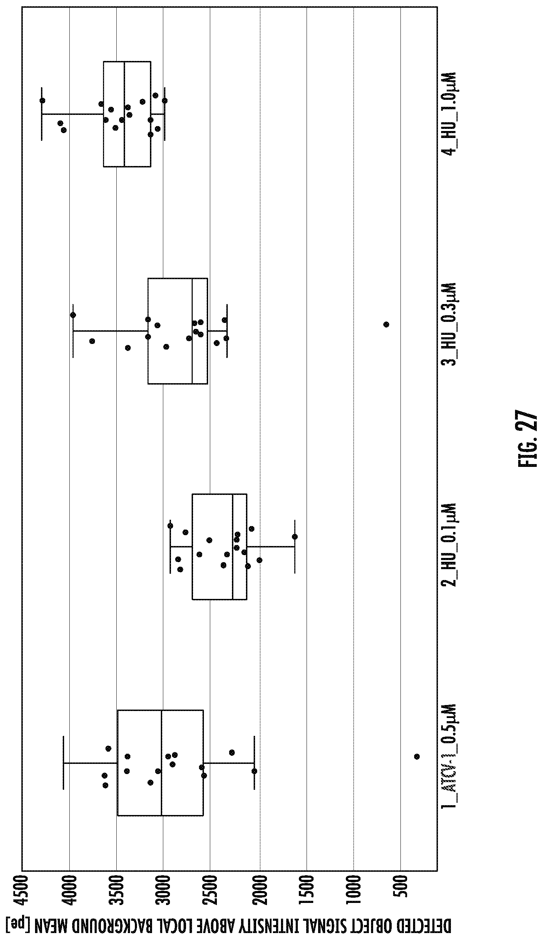

**FIG. 26** show exemplary counts of ligation products from mouse cortex nuclei comparing ATCV-1 (0.5µM) to ATCV-1-HU (0.1µM, 0.3µM or **1.0µM.****FIG. 27** show exemplary signal intensity measurements above background for Satb2 targeted gene ligation products in mouse cortex comparing ATCV-1 and ATCV-1-HU.

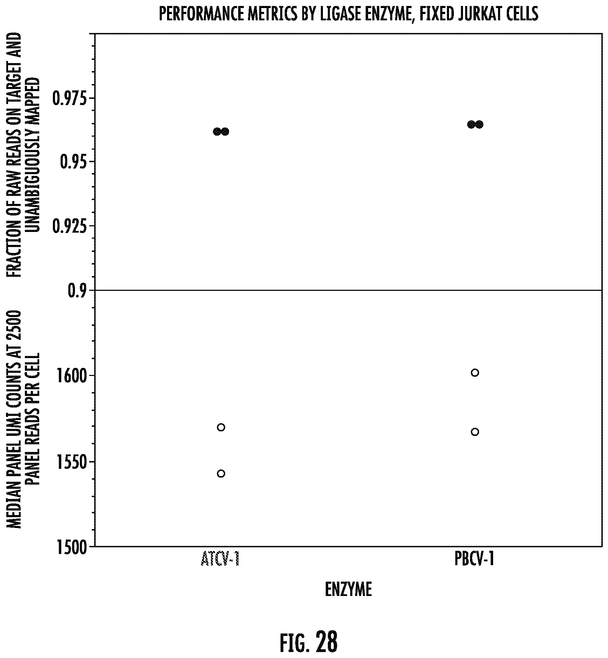

**Fig. 28** is a schematic showing performance metrics for ATCV-1 and PBCV-1 enzymes using a single cell RTL workflow on fixed Jurkat cells. The graph includes the fraction of reads on target and mapped, also referred to as specificity (top), which indicates the percentage of properly paired LHS and RHS probes that have been ligated as well as the median UMI counts per cell at 2,500 panel reads per cell, also referred to as sensitivity (bottom). The UMI counts represent the number of unique RTL probes that have been ligated in each cell by either PBCV-1 (right) or ATCV-1.

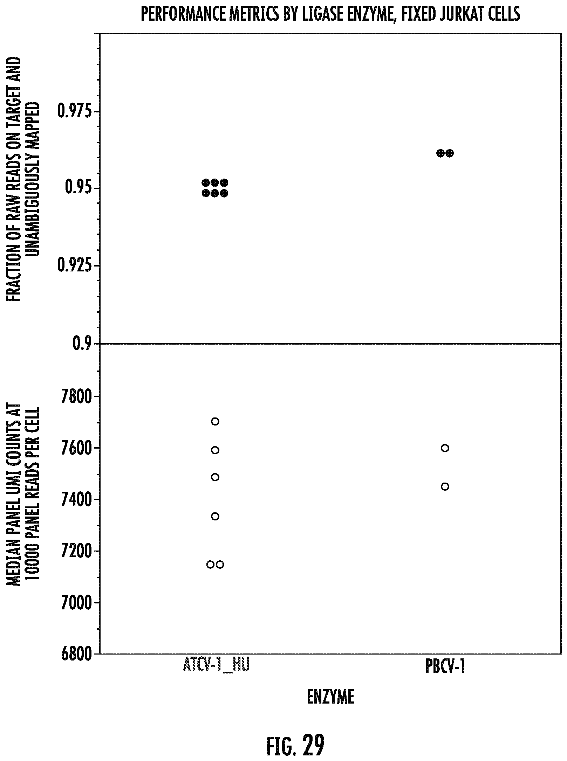

**Fig. 29** is a schematic showing performance metrics for ATCV-HU and PBCV-1 enzymes in a single cell RTL workflow in fixed Jurkat cells. The graph includes the fraction of reads on target and mapped (specificity) (top) which indicates the percentage of properly paired LHS and RHS probes that have been ligated as well as the median UMI counts per cell at 2,500 panel reads per cell (sensitivity) (bottom). The UMI counts represent the number of unique RTL probes that have been ligated in each cell by either PBCV-1 (right) or ATCV-1.

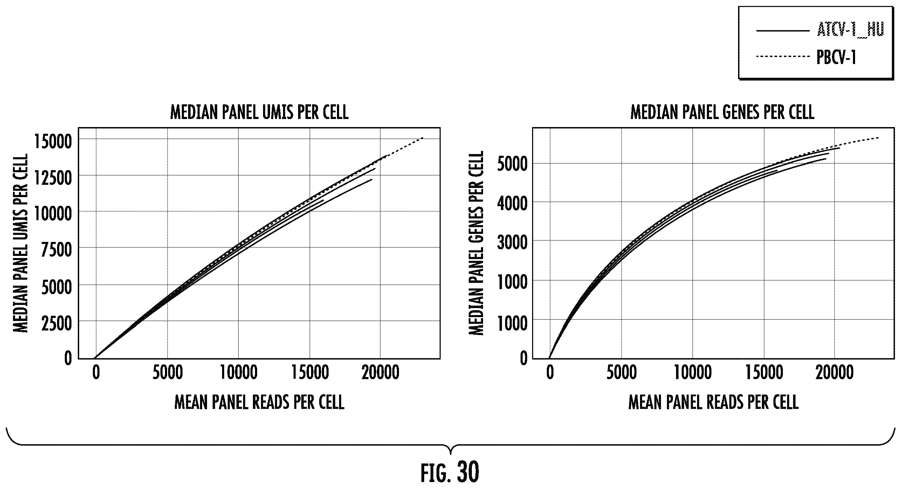

**Fig. 30** depicts overlays of the median UMI's obtained per cell (left) and the median genes obtained per cell (right) detected as a function of sequencing reads mapped to probes targeting the RNA transcripts of interest in fixed Jurkat cells when using either ATCV-1-HU or PBCV-1.

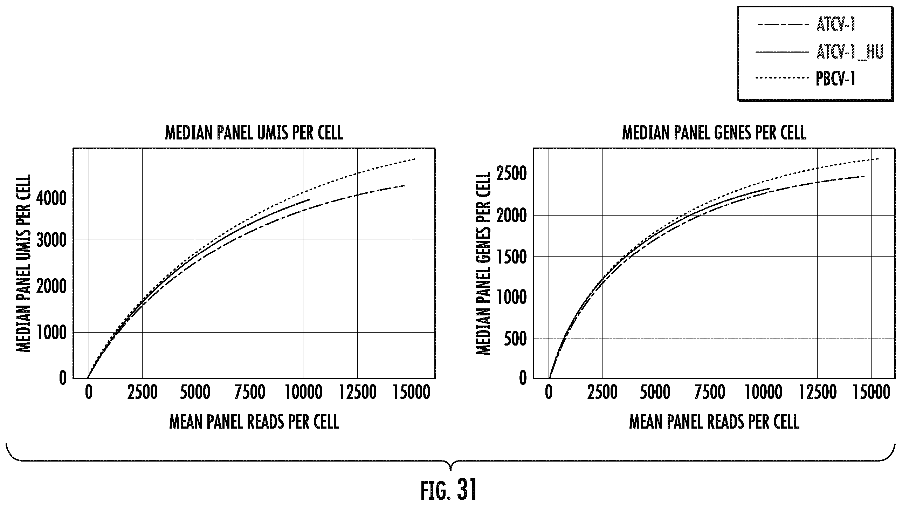

**Fig. 31** depicts overlays of the median UMI's obtained per cell (left) and the median genes obtained per cell (right) detected as a function of sequencing reads mapped to probes targeting the RNA transcripts of interest in fixed PBMCs when using either ATCV-1, ATCV-HU or PBCV-1.

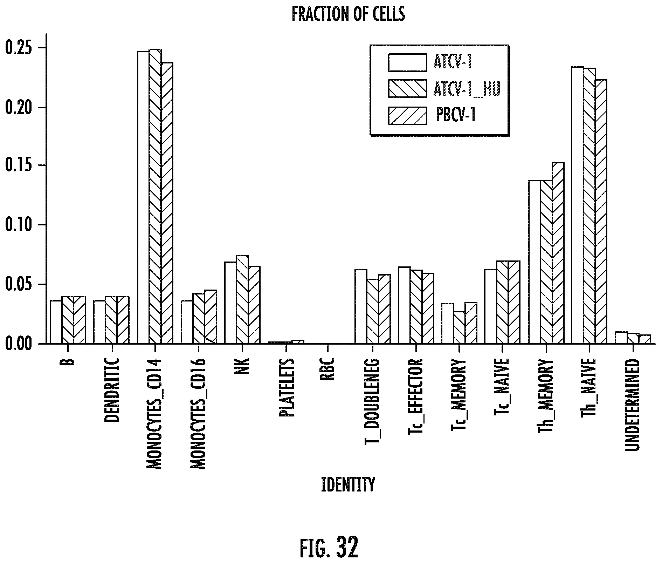

**Fig. 32** provides a chart summarizing the fraction of cells identified as belonging to various subpopulations of cell types present in PBMCs obtained with enzymes (ATCV-1, ATCV-1-HU or PBCV-1. The identity of subpopulations is shown on the x-axis. The three enzymes ligate probes hybridized to RNA transcripts with comparable efficiency such that gene profiles for distinct cell types are preserved. No significant bias in detection of cell subpopulations was observed among the three ligases evaluated.

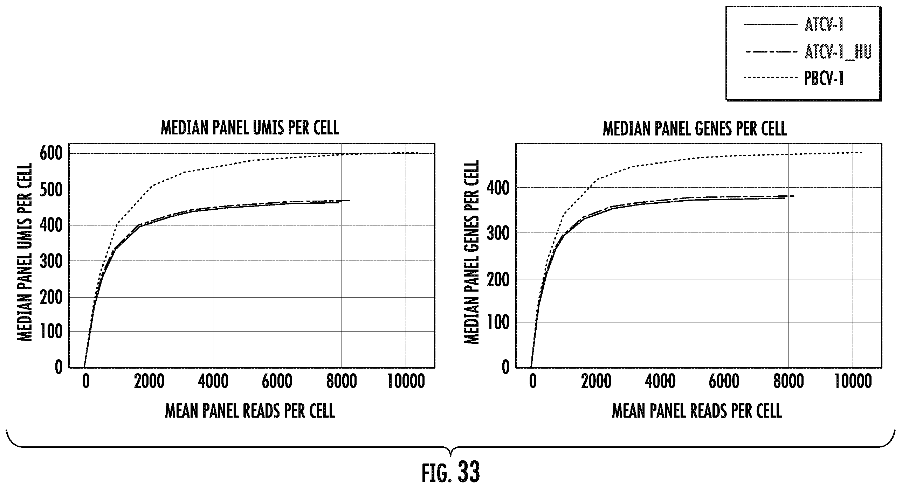

**Fig. 33** depicts overlays of the median UMI's obtained per cell (left) and the median genes obtained per cell (right) detected as a function of sequencing reads mapped to probes targeting the RNA transcripts of interest in fixed dissociated human spleen tissue when using either ATCV-1, ATCV-1-HU or PBCV-1.

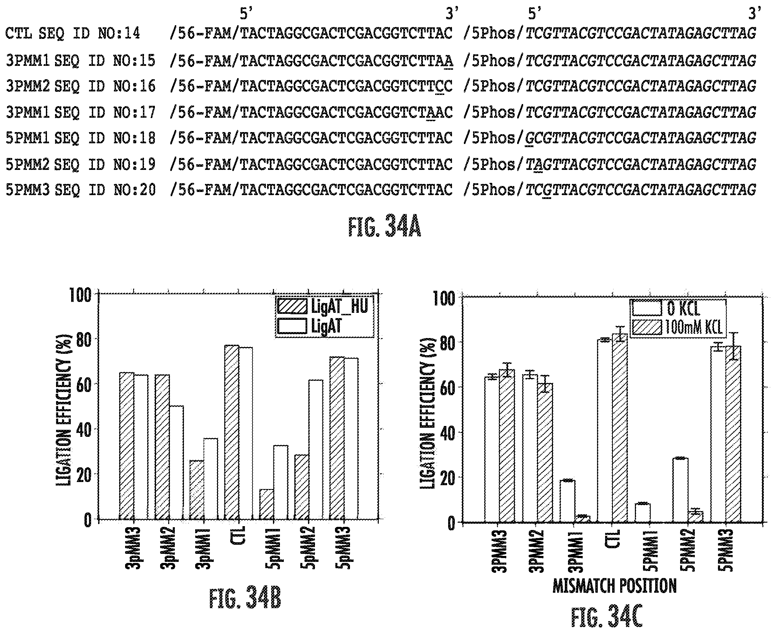

**FIG. 34A, FIG. 34B and FIG.34-C** show **A)** oligonucleotides that were used in ligation experiments for determining the ligation efficiency of ATCV-1 and ATCV-1-HU in the presence of mismatches (e.g., single nucleotide polymorphisms or SNPs). The naming convention is either the first, second or third positional nucleotide at the 3' or 5' end of an oligonucleotide is a mismatch. For example, for 3PMM1, the 3' end of the left side oligonucleotide has a mismatch at the first 3' position (A underlined); 3PMM2, the second nucleotide in from the 3' end of the left side oligonucleotide has the mismatch (C underlined), etc. CTL=control; **B)** an exemplary graph comparing ATCV-1 and ATCV-1-HU for their ability to ligate single base mismatches. X axis is the location of the mismatch, Y axis is the percent ligation efficiency, and **C)** an exemplary graph demonstrating that ATCV-1-HU discrimination of mismatched nucleotides is enhanced in the presence of high salt.

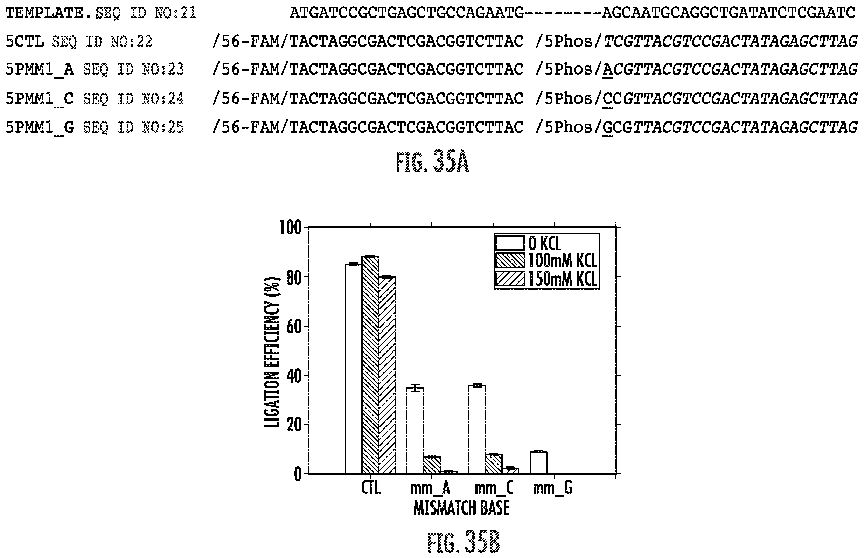

**FIG. 35A and FIG. 35B** shows **A)** oligonucleotides that were used in experiments for determining the ligation efficiency of ATCV-1-HU in discriminating a mismatch at the 5' end of an oligonucleotide. The mismatched nucleotide is underlined at the 5' end of the right side oligonucleotide; and **B)** an exemplary graph demonstrating ATCV-1-HU base bias in discriminating mismatches at the 5' end of an oligonucleotide under different salt conditions.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various altematives to the embodiments of the invention described herein may be employed.

Where values are described as ranges, it will be understood that such disclosure includes all possible sub-ranges within such ranges, as well as specific numerical values that fall within such ranges irrespective of whether a specific numerical value or specific sub-range is expressly stated.

### I. RECOMBINANT LIGASE COMPOSITION

The present disclosure provides a recombinant ligase composition including a recombinantly produced ligase isolated from *Acanthocystis turfacea* chlorella virus 1 ("ATCV-1" or "ATCV1" or "ATCV"), which can additionally be fused to at least one polynucleotide-binding polypeptide. The ATCV-1 ligase is also referred to as "LigAT." The terms "fused" and "operably-linked" and "fusion" may be used interchangeably.

In one embodiment, the ATCV-1 ligase of the recombinant ligase composition comprises an amino acid sequence that is at least 90% identical to the amino acid sequence of SEQ ID NO:1, as follows:

MAIQKPLLAASLKKMSVDNLTFPVYATPKLDGIRALKIDGTLVSRTFKPIRNTTISKVLAS
LLPDGSDGEILSGKTFQDSTSTVMTTDAGIGSDTTFFWFDYVKDDPDKGYLDRIADMKT
FVDQHPEILKDSCVTIVPLFPKKIDTPEELHVFEKWCLDQGFEGVMVRTAGGKYKFGRS
TEKEQILVKIKQFEDDEAVVIGVSALQTNTNDKKLNQLGEMRRTSHQDGKVDLDMLGA

LDVDWNGIRFSIGTGFDKDTREDLWKQRDSIVGKVVKFKYFSQGIKTAPRFPVFLGFRD
ENDM (SEQ ID NO:1)

In other embodiments, the ATCV-1 ligase of the recombinant ligase composition comprises an amino acid sequence that is at least 95% identical or at least 99% identical to the amino acid sequence of SEQ ID NO:1.

The ATCV-1 ligase is specific to *Acanthocystis turfacea* and does not infect or inhabit *Paramecium bursaria.* The ATCV-1 ligase has a predicted molecular weight of approximately 33.71 kDa, and has been confirmed by SDS-PAGE **(****Fig. 12****).** The ATCV-1 ligase can be expressed in soluble form in *Escherichia coli* and purified to homogeneity. Purified ATCV-1 ligase is able to efficiently ligate DNA probes hybridized to an RNA template. Moreover, ATCV-1 ligase's activity and performance are similar to those of the PBCV-1 enzyme after normalizing the concentrations of the two enzymes. PBCV-1 is also known commonly and referred to as SplintR^{®} Ligase (available from New England BioLabs) or Chlorella virus DNA Ligase.

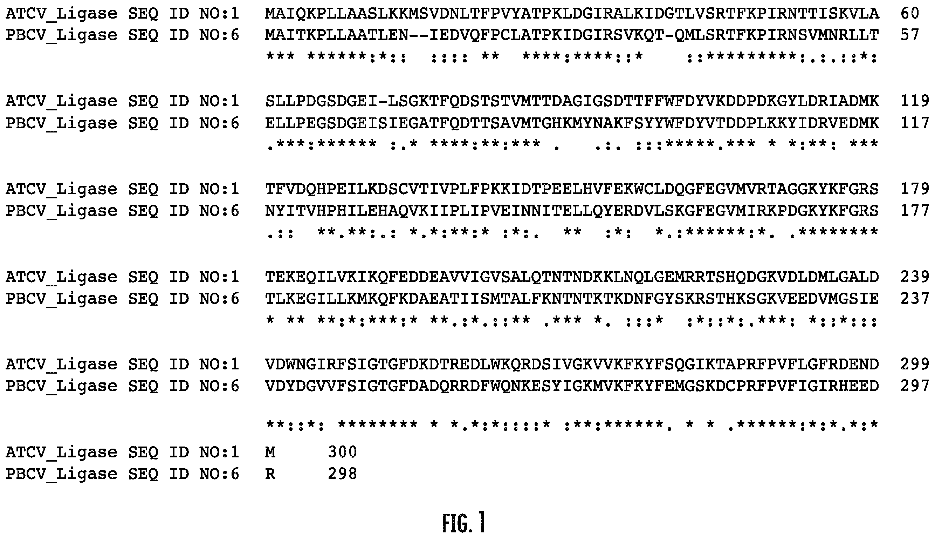

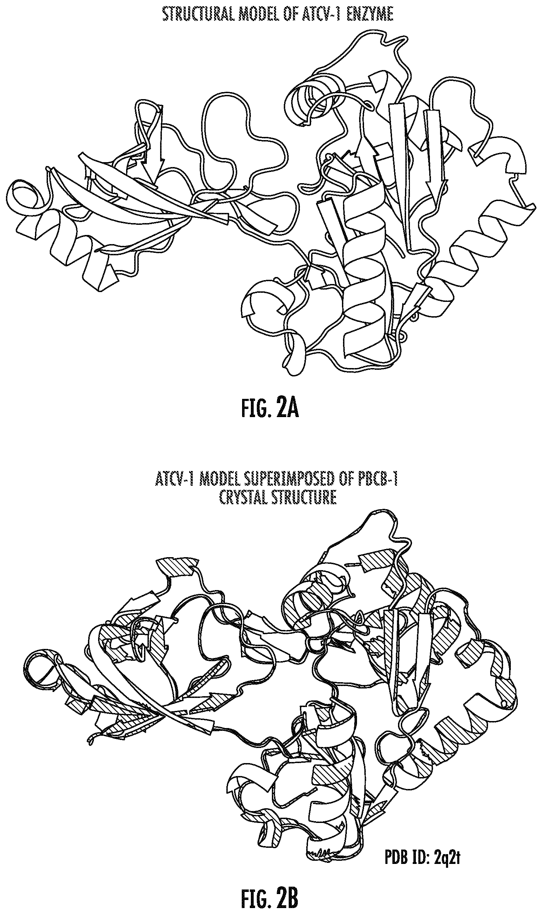

As shown in **FIG. 1****,** based on sequence alignment analyses, the ATCV-1 ligase of the present disclosure shares only approximately 53 percent (52.86%) amino acid sequence identity with PBCV-1 ligase. As shown in **FIG. 2****,** despite their lack of significant sequence homology, there is a high degree of structural similarity based on a comparison of the structural model of ATCV-1 ligase with the crystal structure of PBCV-1 ligase.

The at least one polynucleotide-binding polypeptide portion of the recombinant ligase composition can be a DNA-binding polypeptide or an RNA-binding polypeptide.

In one embodiment, the polynucleotide-binding polypeptide is a HU protein or a functional variant or functional fragment thereof. HU proteins are small, basic, heat-stable DNA-binding protein that are well-conserved in prokaryotes and associated with the bacterial nucleoid. Examples of HU proteins include, without limitation, the following: UniProtKB Entries P0ACF0 (DBHA _ECOLI) (*Escherichia coli*), P0ACF0 (DBHA_ECOLI) (*Escherichia coli*), P0ACF4 (DBHB_ECOLI) (*Escherichia coli*), E0J6W8 (DBHA_ECOLW) (*Escherichia coli*), P0ACF2 (DBHA_ECO57) (*Escherichia coli*), P0ACF1 (DBHA _ECOL6) (*Escherichia coli*), P0A3H0 (DBH_GEOSE) (*Geobacillus stearothermophilus* (*Bacillus stearothermophilus*)), P05514 (DBH_NOSS1) (*Nostoc sp*.), P52680 (DBHA_SERMA) (*Serratia marcescens*), P52681 (DBHB_SERMA) (*Serratia marcescens*), P0ACF3 (DBHA_SHIFL) (*Shigella flexneri*)*,* Q31TZ7 (Q31TZ7_SHIBS) (*Shigella boydii*), and A0A2T4HNA2 (A0A2T4HNA2_MORMO) (*Morganella morganii* (*Proteus morganii*)).

In a particular embodiment, the polynucleotide-binding polypeptide portion of the recombinant ligase fusion composition is the HU protein from *Escherichia coli* of UniProtKB Entry No. P0ACF0 (DBHA_ECOLI), comprising an amino acid sequence of SEQ ID NO:7, as follows:

MNKTQLIDVIAEKAELSKTQAKAALESTLAAITESLKEGDAVQLVGFGTFKVNHRAERT
GRNPQTGKEIKIAAANVPAFVSGKALKDAVK (SEQ ID NO:7)

In other embodiments, the polynucleotide-binding polypeptide portion of the recombinant ligase fusion composition comprises an amino acid sequence that is at least 90% identical, at least 95% identical, or at least 99% identical to the amino acid sequence of SEQ ID NO:7.

In a particular embodiment, the recombinant ligase fusion composition includes an ATCV-1 ligase fused with a HU protein, with this recombinant ligase composition being referred to herein as ATCV-1-HU (also referred to as LigAT_HU or ATCV1-HU).

In a specific embodiment, the ATCV-1-HU recombinant ligase fusion composition comprises a nucleotide sequence of SEQ ID NO:8, as follows:

ATGGCTATTCAGAAGCCGTTACTGGCGGCCAGCCTGAAGAAAATGTCTGTTGACAAC
TTGACTTTCCCGGTCTACGCAACGCCGAAACTGGACGGTATCCGTGCGCTGAAGATT
GACGGCACCCTGGTGTCGCGTACGTTCAAACCGATCCGCAATACTACCATCTCCAAG
GTCCTGGCGAGCTTGCTGCCGGACGGCAGCGATGGTGAGATCTTGTCTGGTAAGACC
TTTCAGGATAGCACCAGCACCGTGATGACGACCGATGCGGGTATCGGTAGCGATAC
CACCTTCTTCTGGTTTGATTACGTTAAAGACGACCCGGATAAGGGTTACCTGGACCG
TATCGCTGACATGAAAACCTTCGTCGACCAGCACCCGGAAATCCTGAAAGACAGCT
GCGTGACGATCGTCCCGCTGTTCCCGAAGAAAATTGACACGCCAGAAGAACTGCAT
GTGTTTGAGAAATGGTGTCTGGACCAGGGTTTTGAGGGCGTTATGGTGCGTACGGCC
GGTGGCAAGTATAAGTTTGGTCGCAGCACCGAGAAAGAACAAATCCTGGTTAAGAT
TAAACAGTTTGAAGATGATGAAGCTGTTGTGATCGGTGTGAGCGCGCTGCAAACCA
ATACGAACGATAAGAAACTGAATCAGCTGGGTGAGATGCGTCGTACTAGCCACCAA
GACGGCAAAGTTGACCTGGACATGCTGGGTGCGCTTGACGTGGACTGGAATGGTAT
TCGTTTCAGCATCGGTACGGGCTTTGATAAAGACACCCGCGAAGATCTCTGGAAACA
ACGCGACAGCATCGTGGGTAAAGTCGTTAAGTTCAAGTATTTCTCCCAGGGTATTAA
GACCGCTCCGCGTTTTCCTGTGTTTCTGGGTTTTCGTGACGAGAACGATATGGGTGG
CAGCGGTGGCAGCATGAATAAGACGCAACTGATTGACGTCATTGCAGAGAAAGCCG

AGTTGAGCAAGACCCAGGCGAAAGCCGCGCTGGAGAGCACGCTGGCAGCGATTACC
GAGAGCCTGAAAGAGGGCGATGCAGTTCAACTGGTTGGCTTCGGCACCTTCAAGGT
CAACCACCGTGCGGAGCGCACCGGTCGCAACCCGCAGACCGGTAAAGAAATCAAGA
TTGCAGCCGCGAACGTGCCGGCGTTCGTCAGCGGTAAGGCACTGAAAGATGCAGTA
AAG (SEQ ID NO:8)

In other embodiments, the ATCV-1 HU recombinant ligase composition comprises a nucleotide sequence that is at least 90% identical, at least 95% identical, or at least 99% identical to the nucleotide sequence of SEQ ID NO:8.

In a specific embodiment, the ATCV-1-HU recombinant ligase composition comprises an amino acid sequence of SEQ ID NO:9:

MAIQKPLLAASLKKMSVDNLTFPVYATPKLDGIRALKIDGTLVSRTFKPIRNTTISKVLAS
LLPDGSDGEILSGKTFQDSTSTVMTTDAGIGSDTTFFWFDYVKDDPDKGYLDRIADMKT
FVDQHPEILKDSCVTIVPLFPKKIDTPEELHVFEKWCLDQGFEGVMVRTAGGKYKFGRS
TEKEQILVKIKQFEDDEAVVIGVSALQTNTNDKKLNQLGEMRRTSHQDGKVDLDMLGA
LDVDWNGIRFSIGTGFDKDTREDLWKQRDSIVGKVVKFKYFSQGIKTAPRFPVFLGFRD
ENDMGGSGGSMNKTQLIDVIAEKAELSKTQAKAALESTLAAITESLKEGDAVQLVGFG
TFKVNHRAERTGRNPQTGKEIKIAAANVPAFVSGKALKDAVK (SEQ ID NO:9)

**In** reference to the amino acid sequence of SEQ ID NO:9, amino acids 1-300 correspond to an ATCV-1 ligase, amino acids 301-306 correspond to a linker (bolded and underlined above), and amino acids 307-396 correspond to a HU protein.

The recombinant ligase composition of the present disclosure is suitable for use as a replacement ligase in all applications for which PBCV-1 ligase and other ligases (e.g., T4 ligase) are used. Certain uses of the PBCV-1 ligase are found in, for example, U.S. Patent Nos. 10,597,650 and 10,597,710. In certain embodiments, the recombinant ligase composition is suitable for use in efficiently ligating DNA probes that are adjacently hybridized to RNA templates.

Furthermore, the ATCV-1 ligase can be fused with other protein partners like DNA binding domains, as well as RNA binding domains, in order to enhance activity and performance.

Nonlimiting applications of use include practicing templated ligation methods, for example, for single-cell RNA-seq (scRNA-seq) technology, e.g., gel bead-in-emulsion ("GEM") technology (10x Genomics Chromium solutions),spatial transcriptomic analysis, (10x Genomics Visium for FFPE solutions) and in situ methods where templated ligation methods are practiced.

Nonlimiting examples of bioorthogonal reactive moieties include "click" chemistry reactive moieties *(e.g.,* with copper or copper-free click chemistry) (Baskin, J., et al. (2007) Proc Natl Acad Sci USA 104(43):16793-16797). Nonlimiting examples of cell permeable monomers or oligomers include amino acids, dipeptides, and sugar (carbohydrate) molecules (e.g., sugar molecules that can be transported through cell membrane transporters but that are not compatible with cell metabolism).

### II. DEFINITIONS:

Unless defined otherwise herein, all technical and scientific terms used herein have the same meaning as commonly understood by one of ordinary skill in the art to which this invention belongs. Singleton, et al.,_Dictionary of Microbiology and Molecular Biology, second ed., John Wiley and Sons, New York (1994), and Hale & Markham, The Harper Collins Dictionary of Biology, Harper Perennial, NY (1991) provide one of skill with a general dictionary of many of the terms used in this invention. Any methods and materials similar or equivalent to those described herein can be used in the practice or testing of the present invention.

Numeric ranges provided herein are inclusive of the numbers defining the range.

"A," "an" and "the" include plural references unless the context clearly dictates otherwise.

The term "each," when used in reference to a collection of items, is intended to identify an individual item in the collection but does not necessarily refer to every item in the collection, unless expressly stated otherwise, or unless the context of the usage clearly indicates otherwise.

An "adaptor," an "adapter," and a "tag" are terms that are used interchangeably in this disclosure, and refer to species that can be coupled to a polynucleotide sequence (in a process referred to as "tagging") using any one of many different techniques including (but not limited to) ligation, hybridization, and tagmentation. Adaptors can also be nucleic acid sequences that add one or more functions, e.g., spacer sequences, primer sequences/sites, barcode sequences, unique molecular identifier sequences.

In general, a sequence element located "at the 3' end" includes the 3'-most nucleotide of the oligonucleotide, and a sequence element located "at the 5' end" includes the 5'-most nucleotide of the oligonucleotide.

A "barcode" is a label, or identifier, that conveys or is capable of conveying information (e.g., information about an analyte in a sample, a bead, and/or a nucleic acid probe). A barcode can be part of an analyte (e.g., nucleic acid molecule), or independent of an analyte. A barcode can be attached to an analyte or attached to a probe bound to an analyte. A nucleic acid probe may comprise a barcode (e.g., a barcode sequence). A particular barcode can be unique relative to other barcodes. A particular set of barcodes can be unique relative to other sets of barcodes.

Barcodes can have a variety of different formats. For example, barcodes can include polynucleotide barcodes, random nucleic acid and/or amino acid sequences, and synthetic nucleic acid and/or amino acid sequences. A barcode can be attached to an analyte or to another moiety or structure (e.g., a probe from a pair of probes) in a reversible or irreversible manner. A barcode can be added to, for example, a fragment of a deoxyribonucleic acid (DNA) or ribonucleic acid (RNA) sample before or during sequencing of the sample. A barcode can be provided as part of a probe that can hybridize to a nucleic acid analyte. Barcodes can allow for identification and/or quantification of individual sequencing-reads (*e.g.,* a barcode can be or can include a unique molecular identifier or "UMI").

Barcodes can spatially-resolve molecular components found in biological samples, for example, at single-cell resolution (*e.g.,* a barcode can be or can include a "spatial barcode"). In some embodiments, a barcode includes both a UMI and a spatial barcode. In some embodiments, a barcode includes two or more sub-barcodes that together function as a single barcode. For example, a polynucleotide barcode can include two or more polynucleotide sequences (e.g., sub-barcodes) that are separated by one or more non-barcode sequences.

The term "base pair" or "bp" as used herein refers to a partnership *(i.e.,* hydrogen bonded pairing) of adenine (A) with thymine (T), or of cytosine (C) with guanine (G) in a double stranded DNA molecule. In some embodiments, a base pair may include A paired with Uracil (U), for example, in a DNA/RNA duplex.

The term "bead," as used herein, generally refers to a particle. The bead may be a solid or semi-solid particle. The bead may be a gel bead. The gel bead may include a polymer matrix (e.g., matrix formed by polymerization or cross-linking). The polymer matrix may include one or more polymers (e.g., polymers having different functional groups or repeat units). Polymers in the polymer matrix may be randomly arranged, such as in random copolymers, and/or have ordered structures, such as in block copolymers. Cross-linking can be via covalent, ionic, or inductive, interactions, or physical entanglement. The bead may be a macromolecule. The bead may be formed of nucleic acid molecules bound together. The bead may be formed via covalent or non-covalent assembly of molecules (e.g., macromolecules), such as monomers or polymers. Such polymers or monomers may be natural or synthetic. Such polymers or monomers may be or include, for example, nucleic acid molecules (e.g., DNA or RNA). The bead may be formed of a polymeric material. The bead may be magnetic or non-magnetic. The bead may be rigid. The bead may be flexible and/or compressible. The bead may be disruptable or dissolvable. The bead may be a solid particle (e.g., a metal-based particle including but not limited to iron oxide, gold or silver) covered with a coating comprising one or more polymers. Such coating may be disruptable or dissolvable.

As used herein, the term "barcoded nucleic acid molecule" generally refers to a nucleic acid molecule that results from, for example, the processing of a nucleic acid barcode molecule with a nucleic acid sequence (e.g., nucleic acid sequence complementary to a nucleic acid primer sequence encompassed by the nucleic acid barcode molecule). The nucleic acid sequence may be a targeted sequence or a non-targeted sequence. The nucleic acid barcode molecule may be coupled to or attached to the nucleic acid molecule comprising the nucleic acid sequence. For example, in the methods and systems described herein, hybridization and reverse transcription of a nucleic acid molecule (e.g., a messenger RNA (mRNA) molecule) of a cell with a nucleic acid barcode molecule (e.g., a nucleic acid barcode molecule containing a barcode sequence and a nucleic acid primer sequence complementary to a nucleic acid sequence of the mRNA molecule) results in a barcoded nucleic acid molecule that has a sequence corresponding to the nucleic acid sequence of the mRNA and the barcode sequence (or a reverse complement thereof). The processing of the nucleic acid molecule comprising the nucleic acid sequence, the nucleic acid barcode molecule, or both, can include a nucleic acid reaction, such as, in non-limiting examples, reverse transcription, nucleic acid extension, ligation, etc. The nucleic acid reaction may be performed prior to, during, or following barcoding of the nucleic acid sequence to generate the barcoded nucleic acid molecule. For example, the nucleic acid molecule comprising the nucleic acid sequence may be subjected to reverse transcription and then be attached to the nucleic acid barcode molecule to generate the barcoded nucleic acid molecule, or the nucleic acid molecule comprising the nucleic acid sequence may be attached to the nucleic acid barcode molecule and subjected to a nucleic acid reaction (e.g., extension, ligation) to generate the barcoded nucleic acid molecule. A barcoded nucleic acid molecule may serve as a template, such as a template polynucleotide, that can be further processed (e.g., amplified) and sequenced to obtain the target nucleic acid sequence. For example, in the methods and systems described herein, a barcoded nucleic acid molecule may be further processed (e.g., amplified) and sequenced to obtain the nucleic acid sequence of the nucleic acid molecule (e.g., mRNA).

In another example, in the methods and systems described herein, hybridization of a pair of probes (or a probe set) to a nucleic acid template (e.g., a messenger RNA (mRNA) molecule) of a cell, wherein one or both of the pair of probes comprises (i) a nucleic acid barcode sequence or (ii) an adapter sequence for attaching a nucleic acid barcode sequence (e.g., through use of a splint oligonucleotide) can provide a nucleic acid hybridization complex (e.g., a full or partial duplex) that can be subjected to conditions to link the pair of probes (e.g., by ligation) and/or to link the adapter sequence to the barcode sequence (e.g., by ligation using the splint oligonucleotide and a ligase of the present disclosure) that results in a barcoded nucleic acid molecule that has a sequence corresponding to the pair of probes that hybridized to the nucleic acid template (or a reverse complement thereof) and the barcode sequence (or a reverse complement thereof). Alternatively, the pair of probes can be ligated to one another while hybridized to the template to form a complex, which comprises a nucleic acid barcode sequence on one or both probes.

A "capture probe" refers to any molecule capable of capturing (directly or indirectly) and/or labelling an analyte (*e.g.,* an analyte of interest) in a biological sample.

In general, a "complement" of a given nucleic acid sequence is a sequence that is fully complementary to and hybridizable to the given sequence. In general, a first sequence that is hybridizable to a second sequence or set of second sequences is specifically or selectively hybridizable to the second sequence or set of second sequences, such that hybridization to the second sequence or set of second sequences is preferred (*e.g.,* thermodynamically more stable under a given set of conditions, such as stringent conditions commonly used in the art) in comparison with hybridization with non-target sequences during a hybridization reaction. Typically, hybridizable sequences share a degree of sequence complementarity over all or a portion of their respective lengths, such as 25%-100% complementarity, including at least about 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, and 100% sequence complementarity.

The term "complementary" herein refers to the broad concept of sequence complementarity in duplex regions of a single polynucleotide strand or between two polynucleotide strands between pairs of nucleotides through base-pairing. It is known that an adenine nucleotide is capable of forming specific hydrogen bonds ("base pairing") with a nucleotide, which is thymine or uracil. Similarly, it is known that a cytosine nucleotide is capable of base pairing with a guanine nucleotide. However, in certain circumstances, hydrogen bonds may also form between other pairs of bases, *e.g.,* between adenine and cytosine, etc. "Essentially complementary" herein refers to sequence complementarity in duplex regions of a single polynucleotide strand or between two polynucleotide strands, for example, wherein the complementarity is less than 100% but is greater than 90%, and retains the stability of the duplex region.

The term "DNA polymerase" includes not only naturally-occurring enzymes but also all modified derivatives thereof, including also derivatives of naturally-occurring DNA polymerase enzymes. For instance, in some embodiments, the DNA polymerase can have been modified to remove 5'-3' exonuclease activity. Sequence-modified derivatives or mutants of DNA polymerase enzymes that can be used include, but are not limited to, mutants that retain at least some of the functional, *e.g.,* DNA polymerase activity of the wild-type sequence. Mutations can affect the activity profile of the enzymes, *e.g.,* enhance or reduce the rate of polymerization, under different reaction conditions, e.g., temperature, template concentration, primer concentration, etc. Mutations or sequence-modifications can also affect the exonuclease activity and/or thermostability of the enzyme.

The term "derived from" encompasses the terms "originated from," "obtained from," "obtainable from," "isolated from," and "created from," and generally indicates that one specified material finds its origin in another specified material or has features that can be described with reference to another specified material.

The terms "detectable label," "optical label," and "label" are used interchangeably herein to refer to a directly or indirectly detectable moiety that is associated with (*e.g.,* conjugated to or bound to) a molecule to be detected, *e.g.,* a capture probe or analyte, or a molecule that binds to a capture probe or analyte. The detectable label can be directly detectable by itself (*e.g.,* radioisotope labels, fluorescent labels, or metal labels) or, in the case of an enzymatic label, can be indirectly detectable, *e.g.,* by catalyzing chemical alterations of a chemical substrate compound or composition, which chemical substrate compound or composition is directly detectable. Detectable labels can be suitable for small scale detection and/or suitable for high-throughput screening. As such, suitable detectable labels include, but are not limited to, radioisotopes, fluorophores, chemiluminescent compounds, bioluminescent compounds, dyes, and metals.

The detectable label can be qualitatively detected (*e.g.,* optically or spectrally), or it can be quantified. Qualitative detection generally includes a detection method in which the existence or presence of the detectable label is confirmed, whereas quantifiable detection generally includes a detection method having a quantifiable (*e.g.,* numerically reportable) value such as an intensity, duration, polarization, and/or other properties. In some embodiments, the detectable label is bound to a feature or to a capture probe associated with a feature. For example, detectably labeled features can include a fluorescent, a colorimetric, or a chemiluminescent label attached to a bead (see, for example, Rajeswari et al., J. Microbiol Methods 139:22-28, 2017, and Forcucci et al., J. Biomed Opt. 10:105010, 2015, the entire contents of each of which are incorporated herein by reference).

In some embodiments, a plurality of detectable labels can be attached to or bound to a feature, capture probe, or composition to be detected. For example, detectable labels can be incorporated during nucleic acid polymerization or amplification (*e.g.,* Cy5^{®}-labelled nucleotides, such as Cy5^{®}-dCTP). Detectable labels can be associated with an oligonucleotide or peptide that is complementary or will associate with a nucleic acid or protein inside or on a cell.

Any suitable detectable label can be used. In some embodiments, the detectable label is a fluorophore. For example, the fluorophore can be from a group that includes: 7-AAD (7-Aminoactinomycin D), Acridine Orange (+DNA), Acridine Orange (+RNA), Alexa Fluor^{®} 350, Alexa Fluor^{®} 430, Alexa Fluor^{®} 488, Alexa Fluor^{®} 532, Alexa Fluor^{®} 546, Alexa Fluor^{®} 555, Alexa Fluor^{®} 568, Alexa Fluor^{®} 594, Alexa Fluor^{®} 633, Alexa Fluor^{®} 647, Alexa Fluor^{®} 660, Alexa Fluor^{®} 680, Alexa Fluor^{®} 700, Alexa Fluor^{®} 750, Allophycocyanin (APC), AMCA / AMCA-X, 7-Aminoactinomycin D (7-AAD), 7- Amino-4-methylcoumarin, 6-Aminoquinoline, Aniline Blue, ANS, APC-Cy7, ATTO-TAG^{™} CBQCA, ATTO-TAG^{™} FQ, Auramine O-Feulgen, BCECF (high pH), BFP (Blue Fluorescent Protein), BFP / GFP FRET, BOBO^{™}-1 / BO-PRO^{™}-1, BOBO^{™}-3 / BO-PRO^{™}-3, BODIPY^{®} FL, BODIPY^{®} TMR, BODIPY^{®} TR-X, BODIPY^{®} 530/550, BODIPY^{®} 558/568, BODIPY^{®} 564/570, BODIPY^{®} 581/591, BODIPY^{®} 630/650-X, BODIPY^{®} 650-665-X, BTC, Calcein, Calcein Blue, Calcium Crimson^{™}, Calcium Green-1^{™}, Calcium Orange^{™}, Calcofluor^{®} White, 5-Carboxyfluoroscein (5-FAM), 5-Carboxynaphthofluoroscein, 6-Carboxyrhodamine 6G, 5-Carboxytetramethylrhodamine (5-TAMRA), Carboxy-X-rhodamine (5-ROX), Cascade Blue^{®}, Cascade Yellow^{™}, CCF2 (GeneBLAzer^{™}), CFP (Cyan Fluorescent Protein), CFP / YFP FRET, Chromomycin A3, Cl-NERF (low pH), CPM, 6-CR 6G, CTC Formazan, Cy2^{®}, Cy3^{®}, Cy3.5^{®}, Cy5^{®}, Cy5.5^{®}, Cy7^{®}Cychrome (PE-Cy5), Dansylamine, Dansyl cadaverine, Dansylchloride, DAPI, Dapoxyl, DCFH, DHR, DiA (4-Di-16-ASP), DiD (DilC18(5)), DIDS, Dil (DilC18(3)), DiO (DiOC18(3)), DiR (DilC18(7)), Di-4 ANEPPS, Di-8 ANEPPS, DM-NERF (4.5-6.5 pH), DsRed (Red Fluorescent Protein), EBFP, ECFP, EGFP, ELF^{®} -97 alcohol, Eosin, Erythrosin, Ethidium bromide, Ethidium homodimer-1 (EthD-1), Europium (III) Chloride, 5-FAM (5-Carboxyfluorescein), Fast Blue, Fluorescein-dT phosphoramidite, FITC, Fluo-3, Fluo-4, FluorX^{®}, Fluoro-Gold^{™} (high pH), Fluoro-Gold^{™} (low pH), Fluoro-Jade, FM^{®} 1-43, Fura-2 (high calcium), Fura-2 / BCECF, Fura Red^{™} (high calcium), Fura Red^{™} / Fluo-3, GeneBLAzer^{™} (CCF2), GFP Red Shifted (rsGFP), GFP Wild Type, GFP / BFP FRET, GFP / DsRed FRET, Hoechst 33342 & 33258, 7-Hydroxy-4-methylcoumarin (pH 9), 1,5 IAEDANS, Indo-1 (high calcium), Indo-1 (low calcium), Indodicarbocyanine, Indotricarbocyanine, JC-1, 6-JOE, JOJO^{™}-1 / JO-PRO^{™}-1, LDS 751 (+DNA), LDS 751 (+RNA), LOLO^{™}-1 / LO-PRO^{™}-1, Lucifer Yellow, LysoSensor^{™} Blue (pH 5), LysoSensor^{™} Green (pH 5), LysoSensor^{™} Yellow/Blue (pH 4.2), LysoTracker^{®} Green, LysoTracker^{®} Red, LysoTracker^{®} Yellow, Mag-Fura-2, Mag-Indo-1, Magnesium Green^{™}, Marina Blue^{®}, 4-Methylumbelliferone, Mithramycin, MitoTracker^{®} Green, MitoTracker^{®} Orange, MitoTracker^{®} Red, NBD (amine), Nile Red, Oregon Green^{®} 488, Oregon Green^{®} 500, Oregon Green^{®} 514, Pacific Blue, PBF1, PE (R-phycoerythrin), PE-Cy5, PE-Cy7, PE-Texas Red, PerCP (Peridinin chlorophyll protein), PerCP-Cy5.5 (TruRed), PharRed (APC-Cy7), C-phycocyanin, R-phycocyanin, R-phycoerythrin (PE), PI (Propidium Iodide), PKH26, PKH67, POPO^{™}-1 / PO-PRO^{™}-1, POPO^{™}-3 / PO-PRO^{™}-3, Propidium Iodide (PI), PyMPO, Pyrene, Pyronin Y, Quantam Red (PE-Cy5), Quinacrine Mustard, R670 (PE-Cy5), Red 613 (PE-Texas Red) , Red Fluorescent Protein (DsRed), Resorufin, RH 414, Rhod-2, Rhodamine B, Rhodamine Green^{™}, Rhodamine Red^{™}, Rhodamine Phalloidin, Rhodamine 110, Rhodamine 123, 5-ROX (carboxy-X-rhodamine), S65A, S65C, S65L, S65T, SBFI, SITS, SNAFL^{®}-1 (high pH), SNAFL^{®}-2, SNARF^{®}-1 (high pH), SNARF^{®}-1 (low pH), Sodium Green^{™}, SpectrumAqua^{®}, SpectrumGreen^{®} #1, SpectrumGreen^{®} #2, SpectrumOrange^{®}, SpectrumRed^{®}, SYTO^{®} 11, SYTO^{®} 13, SYTO^{®} 17, SYTO^{®} 45, SYTOX^{®} Blue, SYTOX^{®} Green, SYTOX^{®} Orange, 5-TAMRA (5-Carboxytetramethylrhodamine), Tetramethylrhodamine (TRITC), Texas Re^{®} / Texas Red^{®}-X, Texas Red^{®}-X (NHS Ester), Thiadicarbocyanine, Thiazole Orange, TOTO^{®}-1 / TO-PRO^{®}-1, TOTO^{®}-3 / TO-PRO^{®}-3, TO-PRO^{®}-5, Tri-color (PE-Cy5), TRITC (Tetramethylrhodamine), TruRed (PerCP-Cy5.5), WW 781, X-Rhodamine (XRITC) , Y66F, Y66H, Y66W, YFP (Yellow Fluorescent Protein), YOYO^{®}-1 / YO-PRO^{®}-1, YOYO^{®}-3 / YO-PRO^{®}-3, 6-FAM (Fluorescein), 6-FAM (NHS Ester), 6-FAM (Azide), HEX, TAMRA (NHS Ester), Yakima Yellow, MAX, TET, TEX615, ATTO 488, ATTO 532, ATTO 550, ATTO 565, ATTO Rho101, ATTO 590, ATTO 633, ATTO 647N, TYE 563, TYE 665, TYE 705, 5' IRDye^{®} 700, 5' IRDye^{®} 800, 5' IRDye^{®} 800CW (NHS Ester), WellRED D4 Dye, WellRED D3 Dye, WellRED D2 Dye, Lightcycler^{®} 640 (NHS Ester), and Dy 750 (NHS Ester).

In some embodiments, a detectable label is or includes a luminescent or chemiluminescent moiety. Common luminescent/chemiluminescent moieties include, but are not limited to, peroxidases such as horseradish peroxidase (HRP), soybean peroxidase (SP), alkaline phosphatase, and luciferase. These protein moieties can catalyze chemiluminescent reactions given the appropriate chemical substrates (*e.g.,* an oxidizing reagent plus a chemiluminescent compound). A number of compound families are known to provide chemiluminescence under a variety of conditions. Non-limiting examples of chemiluminescent compound families include 2,3-dihydro-l,4-phthalazinedione luminol, 5-amino-6,7,8-trimethoxy- and the dimethylamino[ca]benz analog. These compounds can luminesce in the presence of alkaline hydrogen peroxide or calcium hypochlorite and base. Other examples of chemiluminescent compound families include, e.g., 2,4,5-triphenylimidazoles, para-dimethylamino and - methoxy substituents, oxalates such as oxalyl active esters, p-nitrophenyl, N-alkyl acridinium esters, luciferins, lucigenins, or acridinium esters.

The term "duplex" herein refers to a region of complementarity that exists between two polynucleotide sequences. The term "duplex region" refers to the region of sequence complementarity that exists between two oligonucleotides or two portions of a single oligonucleotide. A duplex is a double stranded nucleic acid and may comprise two strands having the same or different length. The duplex may be a fully or partially double stranded nucleic acid duplex. The duplex may be a DNA:DNA duplex, or a DNA:RNA duplex.

An "extendible 3' end" refers an oligonucleotide with a terminal 3' nucleotide that may be extended, for example, by a polymerase enzyme, e.g., a 3' nucleotide that contains a 3' hydroxyl group.

A "gel" refers to a semi-rigid material permeable to liquids and gases.

A "gene" refers to a DNA segment that is involved in producing a polypeptide and includes regions preceding and following the coding regions as well as intervening sequences (introns) between individual coding segments (exons).

A "genome" generally refers to genomic information from a subject, which can be, for example, at least a portion of, or the entirety of, the subject's gene-encoded hereditary information. A genome can include coding regions (*e.g.,* that code for proteins) as well as noncoding regions. A genome can include the sequences of some or all of the subject's chromosomes. For example, the human genome ordinarily has a total of 46 chromosomes. The sequences of some or all of these can constitute the genome.

The terms "hybridizing," "hybridize," "annealing," and "anneal" are used interchangeably in this disclosure, and refer to the pairing of substantially complementary or complementary nucleic acid sequences within two different molecules to form a complex. Pairing can be achieved by any process in which a nucleic acid sequence joins with a substantially or fully complementary sequence through base pairing to form a hybridization complex. For purposes of hybridization, two nucleic acid sequences are "substantially complementary" if at least 60% *(e.g.,* at least 70%, at least 80%, or at least 90%) of their individual bases are complementary to one another.

Herein, "hybridizing nucleotide sequence" refers to a nucleotide sequence, within an oligonucleotide for example, that is capable of hybridizing with a complementary nucleotide sequence in a target nucleic acid molecule present on or within a cell from a tissue sample (e.g., cellular RNA, mRNA). When a hybridizing nucleotide sequence is of such a length that it hybridizes with a complementary, either fully or partially, nucleotide sequence that is unique to a target nucleic acid molecule(s) (e.g., cellular RNA or family of RNAs), the hybridizing nucleotide sequence may be said to hybridize to the same target nucleic acid molecule (e.g., mRNA).

The term "hydrogel" herein refers to a macromolecular polymer gel including a network. Within the network, some polymer chains can optionally be cross-linked, although cross-linking does not always occur.

When referring to immobilization or attachment of molecules (*e.g.,* nucleic acids) to a support, e.g., a solid support, the terms "immobilized" and "attached" are used interchangeably herein, and both terms are intended to encompass direct or indirect, covalent or non-covalent attachment, unless indicated otherwise. In some embodiments, covalent attachment may be preferred, but generally all that is required is that the molecules (*e.g.,* nucleic acids) remain immobilized or attached to the support under the conditions in which it is intended to use the support, for example in nucleic acid amplification and/or sequencing applications.

The terms "isolated," "purified," "separated," and "recovered" as used herein refer to a material (*e.g.,* a protein, nucleic acid, or cell) that is removed from at least one component with which it is naturally associated, for example, at a concentration of at least 90% by weight, or at least 95% by weight, or at least 98% by weight of the sample in which it is contained. For example, these terms may refer to a material which is substantially or essentially free from components which normally accompany it as found in its native state, such as, for example, an intact biological system. An isolated nucleic acid molecule includes a nucleic acid molecule contained in cells that ordinarily express the nucleic acid molecule, but the nucleic acid molecule is present extrachromosomally or at a chromosomal location that is different from its natural chromosomal location.

The terms "linking", "joining" and "ligation" as used herein, with respect to two polynucleotides, such as an adapter oligonucleotide and a sample polynucleotide or a pair of nucleic acid probes (e.g., hybridized to a nucleic acid template), refers to the covalent attachment of two separate polynucleotides to produce a single larger polynucleotide with a contiguous backbone. In some instances, the nucleic acid probes hybridize adjacent on a nucleic acid template. In other instances, the nucleic acid probes hybridize non-adjacently on a nucleic acid template, wherein the 3- end of one of the probes is then extended until it is adjacently positioned to the 5' end of the other probes prior to ligation.

As used herein, a "linker" generally refers to a multifunctional (*e.g.,* bifunctional, trifunctional) reagent used for conjugating two or more chemical moieties.

A "monomer" refers to a molecule that can be covalently bonded to other molecules to form a polymer. An "oligomer" refers to a polymer whose molecules consist of relatively few monomers (*e.g.,* dimer, trimer).

The terms "nucleic acid" and "nucleotide" are intended to be consistent with their use in the art and to include naturally-occurring species or functional analogs thereof. Particularly useful functional analogs of nucleic acids are capable of hybridizing to a nucleic acid in a sequence-specific fashion (*e.g.,* capable of hybridizing to two nucleic acids such that ligation can occur between the two hybridized nucleic acids) or are capable of being used as a template for replication of a particular nucleotide sequence. Naturally-occurring nucleic acids generally have a backbone containing phosphodiester bonds. An analog structure can have an alternate backbone linkage including any of a variety of those known in the art. Naturally-occurring nucleic acids generally have a deoxyribose sugar (*e.g.,* found in deoxyribonucleic acid (DNA)) or a ribose sugar (*e.g.,* found in ribonucleic acid (RNA)).

A nucleic acid can contain nucleotides having any of a variety of analogs of these sugar moieties that are known in the art. A nucleic acid can include native or non-native nucleotides. In this regard, a native deoxyribonucleic acid can have one or more bases selected from the group consisting of adenine (A), thymine (T), cytosine (C), or guanine (G), and a ribonucleic acid can have one or more bases selected from the group consisting of uracil (U), adenine (A), cytosine (C), or guanine (G). Useful non-native bases that can be included in a nucleic acid or nucleotide are known in the art.

A "nucleic acid extension" generally involves incorporation of one or more nucleic acids (e.g., A, G, C, T, U, nucleotide analogs, or derivatives thereof) into a molecule (such as, but not limited to, a nucleic acid sequence) in a template-dependent manner, such that consecutive nucleic acids are incorporated by an enzyme (such as a polymerase or reverse transcriptase), thereby generating a newly synthesized nucleic acid molecule *(i.e.,* an extension product). For example, a primer that hybridizes to a complementary nucleic acid sequence can be used to synthesize a new nucleic acid molecule by using the complementary nucleic acid sequence as a template for nucleic acid synthesis. Similarly, a 3' polyadenylated tail of an mRNA transcript that hybridizes to a poly (dT) sequence (*e.g.,* capture domain) can be used as a template for single-strand synthesis of a corresponding cDNA molecule.

The terms "oligonucleotide" and "polynucleotide" are used interchangeably to refer to a single-stranded multimer of nucleotides from about 2 to about 500 nucleotides in length. Oligonucleotides can be synthetic, or can be made enzymatically (e.g., via polymerization). Oligonucleotides can include ribonucleotide monomers (i.e., can be oligoribonucleotides) and/or deoxyribonucleotide monomers *(i.e.,* oligodeoxyribonucleotides). In some examples, oligonucleotides can include a combination of both deoxyribonucleotide monomers and ribonucleotide monomers in the oligonucleotide (*e.g.,* random or ordered combination of deoxyribonucleotide monomers and ribonucleotide monomers). An oligonucleotide can be 4 to 10, 10 to 20, 21 to 30, 31 to 40, 41 to 50, 51 to 60, 61 to 70, 71 to 80, 80 to 100, 100 to 150, 150 to 200, 200 to 250, 250 to 300, 300 to 350, 350 to 400, or 400-500 nucleotides in length, for example. Oligonucleotides can include one or more functional moieties that are attached (e.g., covalently or non-covalently) to the multimer structure. For example, an oligonucleotide can include one or more detectable labels (e.g., a radioisotope, fluorophore, or metal).

The term "polymerase" herein refers to an enzyme that catalyzes the polymerization of nucleotides (*i*.*e*., the polymerase activity). The term polymerase encompasses DNA polymerases, RNA polymerases, and reverse transcriptases. A "DNA polymerase" catalyzes the polymerization of deoxyribonucleotides. An "RNA polymerase" catalyzes the polymerization of ribonucleotides. A "reverse transcriptase" catalyzes the polymerization of deoxyribonucleotides that are complementary to an RNA template.

As used herein, "polypeptide" refers to a composition comprised of amino acids and recognized as a protein by those of skill in the art. The conventional one-letter or three-letter code for amino acid residues is used herein. The terms "polypeptide" and "protein" are used interchangeably herein to refer to polymers of amino acids of any length. The polymer may be linear or branched, it may include modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

A "primer" is a single-stranded nucleic acid sequence having a 3' end that can be used as a chemical substrate for a nucleic acid polymerase in a nucleic acid extension reaction. RNA primers are formed of RNA nucleotides, and are used in RNA synthesis, while DNA primers are formed of DNA nucleotides and used in DNA synthesis. Primers can also include both RNA nucleotides and DNA nucleotides (*e.g.,* in a random or designed pattern). Primers can also include other natural or synthetic nucleotides described herein that can have additional functionality. In some examples, DNA primers can be used to prime RNA synthesis and vice versa (*e.g.,* RNA primers can be used to prime DNA synthesis). Primers can vary in length. For example, primers can be about 6 bases to about 120 bases. For example, primers can include up to about 25 bases.

A "primer extension" refers to any method where two nucleic acid sequences (*e.g.,* a constant region from each of two distinct capture probes) become linked (*e.g.,* hybridized) by an overlap of their respective terminal complementary nucleic acid sequences *(i.e.,* for example, 3' termini). Such linking can be followed by nucleic acid extension (*e.g.,* an enzymatic extension) of one, or both termini using the other nucleic acid sequence as a template for extension. Enzymatic extension can be performed by an enzyme including, but not limited to, a polymerase and/or a reverse transcriptase.

A "probe", when used in reference to a nucleic acid or sequence of a nucleic acids, is intended as a semantic identifier for the nucleic acid or sequence in the context of a method or composition, and does not limit the structure or function of the nucleic acid or sequence beyond what is expressly indicated. In one example, one or more probes can be hybridized to a nucleic acid template to form a complex (e.g., a duplex) comprising one or more probes and the nucleic acid template.

A "splint oligonucleotide" is an oligonucleotide that, when hybridized to other polynucleotides, acts as a "splint" to position the polynucleotides next to one another so that they can be ligated together. In some embodiments, the splint oligonucleotide is DNA or RNA. The splint oligonucleotide can include a nucleotide sequence that is partially complimentary to nucleotide sequences from two or more different oligonucleotides. In some embodiments, the splint oligonucleotide assists in ligating a "donor" oligonucleotide and an "acceptor" oligonucleotide. In general, an RNA ligase, a DNA ligase, or another other variety of ligase is used to ligate two nucleotide sequences together, for example the ligase of the present disclosure.

In some embodiments, the splint oligonucleotide is between 10 and 50 oligonucleotides in length, e.g., between 10 and 45, 10 and 40, 10 and 35, 10 and 30, 10 and 25, or 10 and 20 oligonucleotides in length. In some embodiments, the splint oligonucleotide is between 15 and 50, 15 and 45, 15 and 40, 15 and 35, 15 and 30, 15 and 30, or 15 and 25 nucleotides in length.

A "subject" or "individual" refers to the source from which a biological sample is obtained, for example, but not limited to, a mammal (*e.g.,* human or a non-human simian), or avian (*e.g.,* bird), or other organism, such as a plant. Examples of subjects include, but are not limited to, a mammal such as a rodent, mouse, rat, rabbit, guinea pig, ungulate, horse, sheep, pig, goat, cow, cat, dog, primate *(i.e.,* human or non-human primate); a plant such as *Arabidopsis thaliana,* corn, sorghum, oat, wheat, rice, canola, or soybean; an algae such as *Chlamydomonas reinhardtii;* a nematode such as *Caenorhabditis elegans;* an insect such as *Drosophila melanogaster,* mosquito, fruit fly, or honey bee; an arachnid such as a spider; a fish such as zebrafish; a reptile; an amphibian such as a frog *or Xenopus laevis; a Dictyostelium discoideum;* a fungi such as *Pneumocystis carinii, Takifugu rubripes,* yeast, *Saccharamoyces cerevisiae* or *Schizosaccharomyces pombe;* or a *Plasmodium falciparum.*

The phrases "substantially similar" and "substantially identical" in the context of at least two nucleic acids typically means that a polynucleotide includes a sequence that has at least about 35%, 40%, 45%, 50%, 55%, 60%, 65%, 70%, 75%, 80%, 85%, 86%, 87%, 88%, 89%, 90%, 91%, 92%, 93%, 94%, 95%, 96%, 97%, 98%, 99%, or even 99.5% sequence identity, in comparison with a reference (e.g., wild-type) polynucleotide or polypeptide. Sequence identity may be determined using known programs such as BLAST, ALIGN, and CLUSTAL using standard parameters. (See, e.g., Altshul et al. (1990) J. Mol. Biol. 215:403-410; Henikoff et al.

(1989) Proc. Natl. Acad. Sci. 89:10915; Karlin et al. (1993) Proc. Natl. Acad. Sci. 90:5873; and Higgins et al. (1988) Gene 73:237). Software for performing BLAST analyses is publicly available through the National Center for Biotechnology Information. Also, databases may be searched using FASTA (Pearson et al. (1988) Proc. Natl. Acad. Sci. 85:2444-2448.) In some embodiments, substantially identical nucleic acid molecules hybridize to each other under stringent conditions *(e.g.,* within a range of medium to high stringency).

A "substrate" is a support that is insoluble in aqueous liquid and which allows for positioning of biological samples, analytes, features, and/or capture probes on the substrate. Further, a "substrate" as used herein, and when not preceded by the modifier "chemical", refers to a member with at least one surface that generally functions to provide physical support for biological samples, analytes, and/or any of the other chemical and/or physical moieties, agents, and structures described herein.

Nucleic acid "synthesis" herein refers to any *in vitro* method for making a new strand of polynucleotide or elongating an existing polynucleotide *(i.e.,* DNA or RNA) in a template dependent manner. Synthesis, according to the invention, can include amplification, which increases the number of copies of a polynucleotide template sequence with the use of a polymerase. Polynucleotide synthesis (*e.g.,* amplification) results in the incorporation of nucleotides into a polynucleotide (*e.g.,* extension from a primer), thereby forming a new polynucleotide molecule complementary to the polynucleotide template. The formed polynucleotide molecule and its template can be used as templates to synthesize additional polynucleotide molecules. "DNA synthesis," as used herein, includes, but is not limited to, polymerase chain reaction (PCR), and may include the use of labeled nucleotides, *e.g.,* for probes and oligonucleotide primers, or for polynucleotide sequencing.

A wide variety of different methods can be used for proximity ligating nucleic acid molecules, including (but not limited to) "sticky-end" and "blunt-end" ligations. Additionally, single-stranded ligation can be used to perform proximity ligation on a single-stranded nucleic acid molecule. Sticky-end proximity ligations involve the hybridization of complementary single-stranded sequences between the two nucleic acid molecules to be joined, prior to the ligation event itself. Blunt-end proximity ligations generally do not include hybridization of complementary regions from each nucleic acid molecule because both nucleic acid molecules lack a single-stranded overhang at the site of ligation.

The term "tag" refers to a detectable moiety that may be one or more atom(s) or molecule(s), or a collection of atoms and molecules. A tag may provide an optical, electrochemical, magnetic, or electrostatic (*e.g.,* inductive, capacitive) signature.

The term "tagged nucleotide" herein refers to a nucleotide that includes a tag (or tag species) that is coupled to any location of the nucleotide including, but not limited to a phosphate (e.g., terminal phosphate), sugar or nitrogenous base moiety of the nucleotide. Tags may be one or more atom(s) or molecule(s), or a collection of atoms and molecules. A tag may provide an optical, electrochemical, magnetic, or electrostatic (*e.g.,* inductive, capacitive) signature.

As used herein, the term "target polynucleotide" refers to a nucleic acid molecule or polynucleotide in a population of nucleic acid molecules having a target sequence to which one or more oligonucleotides are designed to hybridize. In some embodiments, a target sequence uniquely identifies a sequence derived from a sample, such as a particular genomic, mitochondrial, bacterial, viral, or RNA (*e.g.,* mRNA, miRNA, primary miRNA, or pre-miRNA) sequence. In some embodiments, a target sequence is a common sequence shared by multiple different target polynucleotides, such as a common adapter sequence joined to different target polynucleotides. "Target polynucleotide" may be used to refer to a double-stranded nucleic acid molecule that includes a target sequence on one or both strands, or a single-stranded nucleic acid molecule including a target sequence, and may be derived from any source of or process for isolating or generating nucleic acid molecules (e.g., a target polynucleotide may be derived from or originate from a sample and may be referred to as a sample polynucleotide). A target polynucleotide may include one or more *(e.g.,* 1, 2, 3, 4, 5, 6, 7, 8, 9, 10, or more) target sequences, which may be the same or different. In general, different target polynucleotides include different sequences, such as one or more different nucleotides or one or more different target sequences.

The term "template DNA molecule" herein refers to a strand of a nucleic acid from which a complementary nucleic acid strand is synthesized by a DNA polymerase, for example, in a primer extension reaction.

The term "template RNA molecule" herein refers to a strand of a ribonucleic acid (i) from which a complementary nucleic acid strand (cDNA) is synthesized by a DNA polymerase, for example, in a primer extension reaction or (ii) to which two probes are hybridized to serve as a substrate for templated ligation, for example in an RNA templated ligation reaction.

The term "template-dependent manner" refers to a process that involves the template dependent extension of a primer molecule (*e.g.,* DNA synthesis by DNA polymerase). The term "template-dependent manner" typically refers to polynucleotide synthesis of RNA or DNA wherein the sequence of the newly synthesized strand of polynucleotide is dictated by the well-known rules of complementary base pairing (see, for example, Watson, J. D. et al., In: Molecular Biology of the Gene, 4th Ed., W. A. Benjamin, Inc., Menlo Park, Calif. (1987)).

### III. METHODS OF USE

The recombinant ligase composition of the present disclosure can be used as a ligase for any reaction involving the ligation of two nucleic acid sequences. Nonlimiting examples of these uses are described below.

### A. Single Cell Applications for Use with ATCV-1 or Fusions thereof

The present disclosure provides a method of processing a sample that comprises generating a nucleic acid complex (or molecule) comprising linked (e.g., ligated by a ligase described herein) nucleic acid probe molecules. The method may comprise providing a sample comprising a nucleic acid molecule (e.g., a nucleic acid template such as an RNA template molecule) having a first and second target region; a first probe having a (i) first probe sequence that is complementary to the first target region (and optionally (ii) an additional probe sequence); and a second probe having (i) a second probe sequence that is complementary to the second target region (and optionally (ii) an additional probe sequence). In some instances, the first target region and the second target region are adjacent and/or are on the disposed on the same nucleic acid strand. The first and second probe sequences may also comprise first and second reactive moieties, respectively. Upon hybridization of the first probe sequence of the first probe to the first target region of the nucleic acid molecule, and hybridization of the second probe sequence of the second probe to the second target region of the nucleic acid molecule, the two probes may be adjacent to one another. A ligase of the present disclosure, such as ATCV-1 or ATCV-1-HU can be used to hybridize the two adjacent probes together, in the presence or absence of a splint oligonucleotide, thereby generating a hybridization complex which could be referred to as a probe-associated nucleic acid complex. In the case where one or more probes further comprises an additional probe sequence, such as a barcode sequence, the hybridization complex may be referred to as a barcoded probe-associated nucleic acid complex.

The barcoded probe-associated nucleic acid molecule may be subjected to amplification reactions to yield an amplified product comprising the first and second target regions and the barcode sequence or sequences complementary to these sequences. Accordingly, the method may provide amplified products without the use of reverse transcription. One or more processes may be performed within a partition such as a droplet or well. This method of probe hybridization to a target nucleic acid template followed by ligation of the two probes using a ligase of the present disclosure can be useful in single cell transcriptomics as known in the art.

### B. Samples Comprising Nucleic Acids For Use in Methods with ATCV-1 or Fusions thereof

A target nucleic acid in a sample that may be processed and/or analyzed using the methods disclosed herein may be or be comprised in an analyte (e.g., a nucleic acid analyte, such as an RNA transcript).

A sample disclosed herein can be or derived from any biological sample. Methods and compositions disclosed herein may be used for analyzing a biological sample, which may be obtained from a subject using any of a variety of techniques disclosed herein including, but not limited to, biopsy, surgery, and laser capture microscopy (LCM), and generally includes cells and/or other biological material from the subject. In addition to the subjects described above, a biological sample can be obtained from a prokaryote such as a bacterium, an archaea, a virus, or a viroid. A biological sample can also be obtained from non-mammalian organisms (e.g., a plant, an insect, an arachnid, a nematode, a fungus, or an amphibian). A biological sample can also be obtained from a eukaryote, such as a tissue sample, a patient derived organoid (PDO) or patient derived xenograft (PDX). A biological sample from an organism may comprise one or more other organisms or components therefrom. For example, a mammalian tissue section may comprise a prion, a viroid, a virus, a bacterium, a fungus, or components from other organisms, in addition to mammalian cells and non-cellular tissue components. Subjects from which biological samples can be obtained can be healthy or asymptomatic individuals, individuals that have or are suspected of having a disease (e.g., a patient with a disease such as cancer) or a predisposition to a disease, and/or individuals in need of therapy or suspected of needing therapy.

A sample may be a fresh sample, a fresh frozen sample, a biopsy, a tissue, from any organism where a nucleic acid to be analyzed may be present. A sample may be a fixed sample. For example, a sample may comprise a plurality of fixed samples, such as a plurality of fixed cells or fixed nuclei. Alternatively or in addition, a sample may comprise a fixed tissue. Fixation of cell or cellular constituent, or a tissue comprising a plurality of cells or nuclei, may comprise application of a chemical species or chemical stimulus. The term "fixed" as used herein with regard to biological samples generally refers to the state of being preserved from decay and/or degradation. "Fixation" generally refers to a process that results in a fixed sample, and in some instances can include contacting the biomolecules within a biological sample with a fixative (or fixation reagent) for some amount of time, whereby the fixative results in covalent bonding interactions such as crosslinks between biomolecules in the sample. A "fixed biological sample" may generally refer to a biological sample that has been contacted with a fixation reagent or fixative. For example, a formaldehyde-fixed biological sample has been contacted with the fixation reagent formaldehyde. "Fixed cells" or "fixed tissues" refer to cells or tissues that have been in contact with a fixative under conditions sufficient to allow or result in the formation of intra- and inter-molecular covalent crosslinks between biomolecules in the biological sample. Generally, contact of biological sample (e.g., a cell or nucleus) with a fixation reagent (e.g., paraformaldehyde or PFA) results in the formation of intra- and inter-molecular covalent crosslinks between biomolecules in the biological sample. In some cases, the fixation reagent, formaldehyde, may result in covalent aminal crosslinks within RNA, DNA, and/or protein molecules. For example, the widely used fixative reagent, paraformaldehyde or PFA, fixes tissue samples by catalyzing crosslink formation between basic amino acids in proteins, such as lysine and glutamine. Both intra-molecular and inter-molecular crosslinks can form in the protein. These crosslinks can preserve protein secondary structure and also eliminate enzymatic activity in the preserved tissue sample. Examples of fixation reagents include but are not limited to aldehyde fixatives (e.g., formaldehyde, also commonly referred to as "paraformaldehyde," "PFA," and "formalin"; glutaraldehyde; etc.), imidoesters, NHS (N-Hydroxysuccinimide) esters, and the like.

In some embodiments, the fixative or fixation reagent useful for fixing samples is formaldehyde. The term "formaldehyde" when used in the context of a fixative may also refer to "paraformaldehyde" (or "PFA") and "formalin", both of which are terms with specific meanings related to the formaldehyde composition (e.g., formalin is a mixture of formaldehyde and methanol). Thus, a formaldehyde-fixed biological sample may also be referred to as formalin-fixed or PFA-fixed. Protocols and methods for the use of formaldehyde as a fixation reagent to prepare fixed biological samples are well known in the art and can be used in the methods and compositions of the present disclosure. For example, suitable ranges of formaldehyde concentrations for use in preparing a fixed biological sample is 0.1 to 10%, 1-8%, 1-4%, 1-2%, 3-5%, or 3.5-4.5%. In some embodiments of the present disclosure the biological sample is fixed using a final concentration of 1% formaldehyde, 4% formaldehyde, or 10% formaldehyde. Typically, the formaldehyde is diluted from a more concentrated stock solution - e.g., a 35%, 25%, 15%, 10%, 5% PFA stock solution.

Other examples of fixatives include, for example, organic solvents such as alcohols (e.g., methanol or ethanol), ketones (e.g., acetone), and aldehydes (e.g., paraformaldehyde, formaldehyde (e.g., formalin), or glutaraldehyde). As described herein, cross-linking agents may also be used for fixation including, without limitation, disuccinimidyl suberate (DSS), dimethylsuberimidate (DMS), formalin, and dimethyladipimidate (DMA), dithio-bis(-succinimidyl propionate) (DSP), disuccinimidyl tartrate (DST), and ethylene glycol bis(succinimidyl succinate) (EGS). In some cases, a cross-linking agent may be a cleavable cross-linking agent (e.g., thermally cleavable, photocleavable, etc.).

In some cases, more than one fixation reagent can be used in combination when preparing a fixed biological sample. For example, a first fixation agent, such as an organic solvent, may be used in combination with a second fixation agent, such as a cross-linking agent. The organic solvent may be an alcohol (e.g., ethanol or methanol), ketone (e.g., acetone), or aldehyde (e.g., paraformaldehyde, formaldehyde, or glutaraldehyde). The cross-linking agent may be selected from the group consisting of disuccinimidyl suberate (DSS), dimethylsuberimidate (DMS), formalin, and dimethyladipimidate (DMA), dithio-bis(-succinimidyl propionate) (DSP), disuccinimidyl tartrate (DST), and ethylene glycol bis(succinimidyl succinate) (EGS). In some cases, a first fixation agent may be provided to or brought into contact with the cell to bring about a change in a first characteristic or set of characteristics of the cell, and a fixation agent may be provided to or brought into contact with the cell to bring about a change in a second characteristic or set of characteristics of the cell. For example, a first fixation agent may be provided to or brought into contact with a cell to bring about a change in a dimension of the cell (e.g., a reduction in cross-sectional diameter, see, e.g., U.S. Pat. Pub. No. 2020/0033237, which is incorporated herein by reference in its entirety), and a second fixation agent may be provided to or brought into contact with a cell to bring about a change in a second characteristic or set of characteristics of the cell (e.g., forming crosslinks within and/or surrounding the cell). The first and second fixation agents may be provided to or brought into contact with the cell at the same or different times.

In an example, a first fixation agent that is an organic solvent may be provided to a cell to change a first characteristic (e.g., cell size) and a second fixation agent that is a cross-linking agent may be provided to a cell to change a second characteristic (e.g., cell fluidity or rigidity). The first fixation agent may be provided to the cell before the second fixation agent.

Changes to a characteristic or a set of characteristics of a cell or cellular constituents (e.g., incurred upon interaction with one or more fixation agents) may be at least partially reversible (e.g., via rehydration or de-crosslinking). Alternatively, changes to a characteristic or set of characteristics of a cell or cellular constituents (e.g., incurred upon interaction with one or more fixation agents) may be substantially irreversible.

A sample (e.g., a cell sample) may be subjected to a fixation process at any useful point in time. For example, cells and/or cellular constituents of a sample may be subjected to a fixation process involving one or more fixation agents (e.g., as described herein) prior to commencement of any subsequent processing, such as for storage. Cells and/or cellular constituents, such as cells and/or cellular constituents of a tissue sample, subjected to a fixation process prior to storage, may be stored in an aqueous solution, optionally in combination with one or more preserving agents configured to preserve morphology, size, or other features of the cells and/or cellular components. Fixed cells and/or cellular constituents may be stored below room temperature, such as in a freezer. Alternatively, cells and/or cellular constituents of a sample may be subjected to a fixation process involving one or more fixation agents subsequent to one or more other processes, such as filtration, centrifugation, agitation, selective precipitation, purification, permeabilization, isolation, heating, etc. For example, cells, nuclei, and/or cellular constituents of a given type from a sample may be subjected to a fixation process following a separation and/or enrichment procedure (e.g., as described herein). In an example, a sample comprising a plurality of cells including a plurality of cells of a given type may be subjected to a positive separation process to provide a sample enriched in the plurality of cells of the given type. The enriched sample may then be subjected to a fixation process involving one or more fixation agents (e.g., as described herein) to provide an enriched sample comprising a plurality of fixed cells. A fixation process may be performed in a bulk solution. In some cases, fixed samples (e.g., fixed cells, fixed nuclei, and/or cellular constituents) may be partitioned amongst a plurality of partitions (e.g., droplets or wells) and subjected to processing as described elsewhere herein. In some cases, fixed samples may undergo additional processing, such as partial or complete reversal of a fixation process by, for example, rehydration or de-crosslinking, prior to partitioning and any subsequent processing. In some cases, fixed samples may undergo partial or complete reversal of a fixation process within a plurality of partitions (e.g., prior to or concurrent with additional processing described elsewhere herein).

In some cases, a tissue specimen comprising a plurality of cells and/or cellular constituents may be processed to provide formalin-fixed paraffin-embedded (FFPE) tissue. A tissue specimen may be contacted (e.g., saturated) with formalin and then embedded in paraffin wax. FFPE processing may facilitate preservation of a tissue sample (e.g., prior to subsequent processing and analysis). A tissue sample, including an FFPE tissue sample, may additionally or alternatively be subjected to storage in a low-temperature freezer. Cells and/or cellular constituents may be dissociated from a tissue sample (e.g., FFPE tissue sample) prior to undergoing subsequent processing. **In** some cases, individual cells and/or cellular constituents of a tissue sample such as an FFPE tissue sample may be optically detected, labeled, or otherwise processed prior to any such dissociation. Such detection, labeling, or other processing may be performed according to a 2- or 3-dimensional array and optionally according to a pre-determined pattern.

Any sample as previously listed could provide nucleic acids for either *in vivo* or *in vitro* methods where a ligase of the present disclosure could be used. The methods could be practiced within a cell, for example in one cell, or in a tissue which comprises many different cells and cell types. Additionally, nucleic acids from the previously listed samples could be purified from the sample and used in methods comprising a ligase of the present disclosure. The present disclosure is not limited as to the source of nucleic acids which could be used with a ligase of the present disclosure in methods where ligation as described herein is desired.

### C. Spatial Analysis Applications Using ATCV-1 or Fusions thereof

Cells within a tissue have differences in cell morphology and/or function due to varied analyte levels (e.g., gene and/or protein expression) within the different cells. The specific position of a cell within a tissue (e.g., the cell's position relative to neighboring cells or the cell's position relative to the tissue microenvironment) can affect, e.g., the cell's morphology, differentiation, fate, viability, proliferation, behavior, signaling, and cross-talk with other cells in the tissue.

Spatial heterogeneity has been previously studied using techniques that typically provide data for a handful of analytes in the context of intact tissue or a portion of a tissue (e.g., tissue section), or provide significant analyte data from individual, single cells, but fails to provide information regarding the position of the single cells from the originating biological sample (e.g., tissue).

Spatial analysis methodologies and compositions described herein can provide a vast amount of analyte and/or expression data for a variety of analytes within a biological sample at high spatial resolution, while retaining native spatial context. Spatial analysis methods and compositions can include, e.g., the use of a capture probe including a spatial barcode (e.g., a nucleic acid sequence that provides information as to the location or position of an analyte within a cell or a tissue sample (e.g., mammalian cell or a mammalian tissue sample) and a capture domain that is capable of binding to an analyte (e.g., a protein and/or a nucleic acid) produced by and/or present in a cell. Spatial analysis methods and compositions can also include the use of a capture probe having a capture domain that captures an intermediate agent for indirect detection of an analyte. For example, the intermediate agent can include a nucleic acid sequence (e.g., a barcode) associated with the intermediate agent. Detection of the intermediate agent is therefore indicative of the analyte in the cell or tissue sample.

Non-limiting aspects of spatial analysis methodologies and compositions are described in U.S. Patent Nos. 10,774,374, 10,724,078, 10,480,022, 10,059,990, 10,041,949, 10,002,316, 9,879,313, 9,783,841, 9,727,810, 9,593,365, 8,951,726, 8,604,182, 7,709,198, U.S. Patent Application Publication Nos. 2020/239946, 2020/080136, 2020/0277663, 2020/024641, 2019/330617, 2019/264268, 2020/256867, 2020/224244, 2019/194709, 2019/161796, 2019/085383, 2019/055594, 2018/216161, 2018/051322, 2018/0245142, 2017/241911, 2017/089811, 2017/067096, 2017/029875, 2017/0016053, 2016/108458, 2015/000854, 2013/171621, WO 2018/091676, WO 2020/123305, WO 2020/176788, Rodriques et al., Science 363(6434):1463-1467, 2019; Lee et al., Nat. Protoc. 10(3):442-458, 2015; Trejo et al., PLoS ONE 14(2):e0212031, 2019; Chen et al., Science 348(6233):aaa6090, 2015; Gao et al., BMC Biol. 15:50, 2017; and Gupta et al., Nature Biotechnol. 36:1197-1202, 2018; the Visium Spatial Gene Expression Reagent Kits User Guide (e.g., Rev C, dated June 2020), and/or the Visium Spatial Tissue Optimization Reagent Kits User Guide (e.g., Rev C, dated July 2020), both of which are available at the 10x Genomics Support Documentation website, and can be used herein in any combination. Further non-limiting aspects of spatial analysis methodologies and compositions are described herein.

Some general terminology that may be used in this disclosure can be found in Section (I)(b) of WO 2020/176788 and/or U.S. Patent Application Publication No. 2020/0277663. Typically, a "barcode" is a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes. For the purpose of this disclosure, an "analyte" can include any biological substance, structure, moiety, or component to be analyzed. The term "target" can similarly refer to an analyte of interest.

Analytes can be broadly classified into one of two groups: nucleic acid analytes, and non-nucleic acid analytes. Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral proteins (e.g., viral capsid, viral envelope, viral coat, viral accessory, viral glycoproteins, viral spike, etc.), extracellular and intracellular proteins, antibodies, and antigen binding fragments. In some embodiments, the analyte(s) can be localized to subcellular location(s), including, for example, organelles, e.g., mitochondria, Golgi apparatus, endoplasmic reticulum, chloroplasts, endocytic vesicles, exocytic vesicles, vacuoles, lysosomes, etc. In some embodiments, analyte(s) can be peptides or proteins, including without limitation antibodies and enzymes. Additional examples of analytes can be found in Section (I)(c) of WO 2020/176788 and/or U.S. Patent Application Publication No. 2020/0277663. In some embodiments, an analyte can be detected indirectly, such as through detection of an intermediate agent, for example, a ligation product or an analyte capture agent (e.g., an oligonucleotide-conjugated antibody), such as those described herein. Ligation products can be generated using a ligase isolated *from Acanthocystis turfacea* chlorella virus 1 (ATCV-1) (e.g., a recombinantly produced ATCV-1 ligase), as further described herein.

A "biological sample" is typically obtained from the subject for analysis using any of a variety of techniques including, but not limited to, biopsy, surgery, and laser capture microscopy (LCM), and generally includes cells and/or other biological material from the subject. In some embodiments, a biological sample can be a tissue section. In some embodiments, a biological sample can be a fixed and/or stained biological sample (e.g., a fixed and/or stained tissue section). Non-limiting examples of stains include histological stains (e.g., hematoxylin and/or eosin) and immunological stains (e.g., fluorescent stains). In some embodiments, a biological sample (e.g., a fixed and/or stained biological sample) can be imaged. Biological samples are also described in Section (I)(d) of WO 2020/176788 and/or U.S. Patent Application Publication No. 2020/0277663.

In some embodiments, a biological sample is permeabilized with one or more permeabilization reagents. For example, permeabilization of a biological sample can facilitate analyte capture. Exemplary permeabilization agents and conditions are described in Section (I)(d)(ii)(13) or the Exemplary Embodiments Section of WO 2020/176788 and/or U.S. Patent Application Publication No. 2020/0277663.

Array-based spatial analysis methods involve the transfer of one or more analytes from a biological sample to an array of features on a substrate, where each feature is associated with a unique spatial location on the array. Subsequent analysis of the transferred analytes includes determining the identity of the analytes and the spatial location of the analytes within the biological sample. The spatial location of an analyte within the biological sample is determined based on the feature to which the analyte is bound (e.g., directly or indirectly) on the array, and the feature's relative spatial location within the array.

In some embodiments, more than one analyte type (e.g., nucleic acids and proteins) from a biological sample can be detected (e.g., simultaneously or sequentially) using any appropriate multiplexing technique, such as those described in Section (IV) of WO 2020/176788 and/or U.S. Patent Application Publication No. 2020/0277663.

There are at least two methods to associate a spatial barcode with one or more neighboring cells, such that the spatial barcode identifies the one or more cells, and/or contents of the one or more cells, as associated with a particular spatial location. One method is to promote analytes or analyte proxies (e.g., intermediate agents) out of a cell and towards a spatially-barcoded array (e.g., including spatially-barcoded capture probes). Another method is to cleave spatially-barcoded capture probes from an array and promote the spatially-barcoded capture probes towards and/or into or onto the biological sample.

In some cases, targeted probes may be configured to form ligation products while hybridized to a template (e.g., a DNA or RNA template, such as an analyte or an intermediate agent, or portion thereof), thereby creating ligations products that serve as proxies for a template. Ligation products can be generated using a ligase isolated from *Acanthocystis turfacea* chlorella virus 1 (ATCV-1) (e.g., a recombinantly produced ATCV-1 ligase) and/or a recombinant ligase or fusion composition, as further described herein.

Ligation products can be generated using a ligase isolated *from Acanthocystis turfacea* chlorella virus 1 (ATCV-1) (e.g., a recombinantly produced ATCV-1 ligase) and/or a recombinant ligase fusion composition, as further described herein.

Spatial information can provide information of biological and/or medical importance. For example, the methods and compositions described herein can allow for: identification of one or more biomarkers (e.g., diagnostic, prognostic, and/or for determination of efficacy of a treatment) of a disease or disorder; identification of a candidate drug target for treatment of a disease or disorder; identification (e.g., diagnosis) of a subject as having a disease or disorder; identification of stage and/or prognosis of a disease or disorder in a subject; identification of a subject as having an increased likelihood of developing a disease or disorder; monitoring of progression of a disease or disorder in a subject; determination of efficacy of a treatment of a disease or disorder in a subject; identification of a patient subpopulation for which a treatment is effective for a disease or disorder; modification of a treatment of a subject with a disease or disorder; selection of a subject for participation in a clinical trial; and/or selection of a treatment for a subject with a disease or disorder.

Spatial information can provide information of biological importance. For example, the methods and compositions described herein can allow for: identification of transcriptome and/or proteome expression profiles (e.g., in healthy and/or diseased tissue); identification of multiple analyte types in close proximity (e.g., nearest neighbor analysis); determination of up- and/or down-regulated genes and/or proteins in diseased tissue; characterization of tumor microenvironments; characterization of tumor immune responses; characterization of cells types and their co-localization in tissue; and identification of genetic variants within tissues (e.g., based on gene and/or protein expression profiles associated with specific disease or disorder biomarkers).

Generally, analytes and/or intermediate agents (or portions thereof) can be captured when contacting a biological sample with a substrate including capture probes (e.g., a substrate with capture probes embedded, spotted, printed, fabricated on the substrate, or a substrate with features (e.g., beads, wells) comprising capture probes). As used herein, "contact," "contacted," and/or "contacting," a biological sample with a substrate refers to any contact (e.g., direct or indirect) such that capture probes can interact (e.g., bind covalently or non-covalently (e.g., hybridize)) with analytes from the biological sample. Capture can be achieved actively (e.g., using electrophoresis) or passively (e.g., using diffusion). Analyte capture is further described in Section (II)(e) of WO 2020/176788 and/or U.S. Patent Application Publication No. 2020/0277663.

In some cases, spatial analysis can be performed by detecting multiple oligonucleotides that hybridize to an analyte. In some instances, for example, spatial analysis can be performed using RNA-templated ligation (RTL). Methods of RTL have been described previously. See, e.g., Credle et al., Nucleic Acids Res. 2017 Aug 21;45(14):e128. Typically, RTL includes hybridization of two oligonucleotides to adjacent sequences on an analyte (e.g., an RNA molecule, such as an mRNA molecule). In some instances, the oligonucleotides are DNA molecules. In some instances, one of the oligonucleotides includes at least two ribonucleic acid bases at the 3' end and/or the other oligonucleotide includes a phosphorylated nucleotide at the 5' end. In some instances, one of the two oligonucleotides includes a capture domain (e.g., a poly(A) sequence, a non-homopolymeric sequence). After hybridization to the analyte, a ligase (e.g., a ligase isolated *from Acanthocystis turfacea* chlorella virus 1 (ATCV-1)), such as a recombinantly produced ATCV-1 ligase, and/or a recombinant ligase composition, as further described herein) ligates the two oligonucleotides together, creating a ligation product. In some instances, the two oligonucleotides hybridize to sequences that are not adjacent to one another. For example, hybridization of the two oligonucleotides creates a gap between the hybridized oligonucleotides. In some instances, a polymerase (e.g., a DNA polymerase) can extend one of the oligonucleotides prior to ligation. After ligation, the ligation product is released from the analyte. In some instances, the ligation product is released using an endonuclease (e.g., RNAse H). The released ligation product can then be captured by capture probes (e.g., instead of direct capture of an analyte) on an array, optionally amplified, and sequenced, thus determining the location and optionally the abundance of the analyte in the biological sample.

During analysis of spatial information, sequence information for a spatial barcode associated with an analyte is obtained, and the sequence information can be used to provide information about the spatial distribution of the analyte in the biological sample. Various methods can be used to obtain the spatial information. In some embodiments, specific capture probes and the analytes they capture are associated with specific locations in an array of features on a substrate. For example, specific spatial barcodes can be associated with specific array locations prior to array fabrication, and the sequences of the spatial barcodes can be stored (e.g., in a database) along with specific array location information, so that each spatial barcode uniquely maps to a particular array location.

Additional compositions, systems, kits, and methods for templated ligation (e.g., using an RNA template) workflows using a spatially barcoded array are provided in PCT/US20/66720, which is incorporated herein by reference in its entirety.

In certain embodiments, the recombinant ligase composition of the present disclosure can be used to ligate a splinted event for spatial analysis. For example, when building an array that has different capture domains (but that retains the barcodes that are pre-ordered on the array), a splinted event can be used to ligate the different capture domains onto the oligo affixed to the arrays. Generating capture probes for spatial analysis are described in WO 2020/123305, with examples of the aforementioned splinted events being shown in Fig. 24 and Fig. 44 of WO 2020/123305.

### D. In situ Applications for use with ATCV-1 or Fusions thereof

In some embodiments, sequence analysis can be performed *in situ. In situ* sequence analysis typically involves detection of labeled nucleotides or oligonucleotides (e.g., fluorescently labeled mononucleotides or dinucleotides) in a sequential, template-dependent manner or hybridization of a labeled oligonucleotide (e.g., a labeled random hexamer) to a nucleic acid template such that the identities (i.e., nucleotide sequence) of the incorporated nucleotides or labeled primer extension products can be determined, and consequently, the nucleotide sequence of the corresponding template nucleic acid. Aspects of in situ sequence analysis are described, for example, in Mitra et al., (2003) Anal. Biochem. 320, 55-65, and Lee et al., (2014) Science, 343(6177), 1360-1363, the entire contents of each of which are incorporated herein by reference.

In addition, examples of methods and systems for performing *in situ* sequencing and analysis are described in PCT Patent Application Publication Nos. WO2014/163886, WO2018/045181, WO2018/045186, U.S. Patent Application Publication Nos US2019/0177718, US2019/0194709, and in U.S. Patent Nos. 10,138,509 and 10,179,932, the entire contents of each of which are incorporated herein by reference. Exemplary techniques for *in situ* sequence analysis include, but are not limited to, STARmap (described for example in Wang et al., (2018) Science, 361(6499) 5691), MERFISH (described for example in Moffitt, (2016) Methods in Enzymology, 572, 1-49), and FISSEQ (described for example in U.S. Patent Application Publication No. 2019/0032121). The entire contents of each of the foregoing references are incorporated herein by reference.

The ligated padlock or circular probes disclosed herein can be used with a variety of techniques, including multiplexed *in situ* hybridization or *in situ* sequencing technology of an intact tissue or non-homogenized tissue. In some embodiments, the target nucleic acid is in a cell in a tissue. In some embodiments the tissue has been fixed and permeabilized as described elsewhere herein. In some embodiments, the methods provided herein comprises one or more post-fixing (also referred to as post-fixation) steps. In some embodiments, one or more post-fixing step is performed after contacting a sample with a polynucleotide disclosed herein, e.g., one or more probes such as a circular or padlock probe. In some embodiments, one or more post-fixing step is performed after a hybridization complex comprising a probe and a target is formed in a sample. In some embodiments, one or more post-fixing step is performed prior to a ligation reaction disclosed herein, such as the ligation to circularize a padlock probe. A post-fixing step may be performed using any suitable fixation reagent disclosed herein, for example, 3% (w/v) paraformaldehyde in DEPC-PBS.

In some aspects, provided herein are methods, compositions, kits, devices, and systems for in situ analysis, such as in situ RNA analysis. In some embodiments, the present disclosure provides methods for detecting multiple targets, e.g., transcripts (e.g., the transcriptome or a subset thereof) in a cell or a tissue sample through a nucleic acid probe scheme that includes ligation using a nucleic acid template (e.g., an RNA template). In one embodiment, the ligation is performed using a ligase isolated *from Acanthocystis turfacea* chlorella virus 1 (ATCV-1) (e.g., a recombinantly produced ATCV-1 ligase) and/or a recombinant ligase composition, as further described herein.

In some embodiments, disclosed herein is a method for analyzing a target nucleic acid (e.g., an RNA such as an mRNA transcript of a gene of interest) in a sample, comprising contacting a sample comprising a target nucleic acid with a targeting probe (e.g., a primary probe such as a padlock probe) comprising (1) a targeting region that hybridizes to the target nucleic acid directly or indirectly, and (2) an optional target barcode region that identifies the primary probe. In some embodiments, the targeting probe comprises or is capable of forming a circular probe when hybridized to the target nucleic acid directly or indirectly. In some embodiments, the targeting probe is a linear probe prior to hybridization to the target nucleic acid and can be circularized upon binding to the target nucleic acid. In one embodiment, the linear probe is a padlock probe that hybridizes to an RNA template and is circularized using a ligase isolated from *Acanthocystis turfacea* chlorella virus 1 (ATCV-1) (e.g., a recombinantly produced ATCV-1 ligase), and/or a recombinant ligase composition, as further described herein.

Traditional padlock probes comprise a linear DNA probe where the terminal ends of the probe are complementary to an internal sequence of a target molecule of interest. The nature of the complementarity brings the 5' and 3'-ends of the probe sequence adjacent to each other such that the ends may be ligated to form a circle. One drawback of traditional padlock probes assembled by ligation occurs when the target nucleic acid of interest is a ribonucleic acid (RNA) molecule, such as an mRNA molecule. There is need for in situ transcriptomic tools for the spatial mapping of gene expression within tissues at cellular, or even subcellular resolution, including multiplexed in situ RNA hybridization and sequencing techniques.

Rolling circle amplification of RNA sequences using padlock probes allows for amplification of the target sequence in a highly quantitative manner without relying on thermocyclers or other advanced read-out systems. However, the assembly of padlock probes which are deoxynucleic acids (DNA) directly on an RNA template (e.g., without converting an RNA to cDNA) requires the use of an RNA-templated ligase to close the circle of a linear DNA probe to circularize the padlock. This ligation event is inefficient. While this efficiency can potentially be increased through the incorporation of ribonucleotides into DNA padlock probes, this requires the use of specialized RNA ligase enzymes (e.g., PBCV-1 ligase) and can significantly increase the cost of manufacturing padlock probes, especially for multiplexed assays utilizing libraries of padlock probes. These drawbacks can be addressed by the methods, systems, compositions, and kits described herein.

In some embodiments, the analysis of nucleic acids described herein comprises generation of a hybridization product comprising the pairing of substantially complementary or complementary nucleic acid sequences within two different molecules, e.g., an analyte and a probe, such as a padlock probe. Pairing can be achieved by any process in which a nucleic acid sequence joins with a substantially or fully complementary sequence through base pairing to form a hybridization complex. For purposes of hybridization, two nucleic acid sequences are "substantially complementary" if at least 60% (e.g., at least 70%, at least 80%, or at least 90%) of their individual bases are complementary to one another. Various probes and probe sets can be hybridized to an analyte and each probe may comprise one or more barcode sequences. Exemplary barcoded probes or probe sets may be based on, e.g., a padlock probe.

In some embodiments, the analysis of nucleic acids described herein comprises ligating one or more probes using the ATCV-1 ligases and/or the recombinant ligase fusion composition disclosed herein such as the ligation of a padlock probe to generate a circular molecule, or the ligation of two adjacently hybridized probes.

In some embodiments, the ligation herein is a direct ligation. In some embodiments, the ligation herein is an indirect ligation. "Direct ligation" means that the ends of the polynucleotides hybridize immediately adjacently to one another to form a substrate for a ligase enzyme resulting in their ligation to each other (intramolecular ligation). Alternatively, "indirect" means that the ends of the polynucleotides hybridize non-adjacently to one another, e.g., separated by one or more intervening nucleotides or "gaps". In some embodiments, said ends are not ligated directly to each other, but instead occurs either via the intermediacy of one or more intervening (so-called "gap" or "gap-filling" (oligo)nucleotides) or by the extension of the 3' end of a probe to "fill" the "gap" corresponding to said intervening nucleotides (intermolecular ligation). In some cases, the gap of one or more nucleotides between the hybridized ends of the polynucleotides may be "filled" by one or more "gap" (oligo)nucleotide(s) which are complementary to a splint, padlock probe, or target nucleic acid. The gap may be a gap of 1 to 60 nucleotides or a gap of 1 to 40 nucleotides or a gap of 3 to 40 nucleotides. In specific embodiments, the gap may be a gap of about 1, 2, 3, 4, 5, 6, 7, 8, 9 or 10 or more nucleotides, of any integer (or range of integers) of nucleotides in between the indicated values. In some embodiments, the gap between said terminal regions may be filled by a gap oligonucleotide or by extending the 3' end of a polynucleotide. In some cases, ligation involves ligating the ends of the probe to at least one gap (oligo)nucleotide, such that the gap (oligo)nucleotide becomes incorporated into the resulting polynucleotide. In some embodiments, the ligation herein is preceded by gap filling. In other embodiments, the ligation herein does not require gap filling.

In some embodiments, ligation of the polynucleotides produces polynucleotides with melting temperature higher than that of unligated polynucleotides. Thus, in some aspects, ligation stabilizes the hybridization complex containing the ligated polynucleotides prior to subsequent steps, comprising amplification and detection.

The following examples are intended to illustrate, but not limit, the invention.

### EXAMPLES

### Example 1

### Assay for DNA Oligonucleotide Ligation using RNA Splint

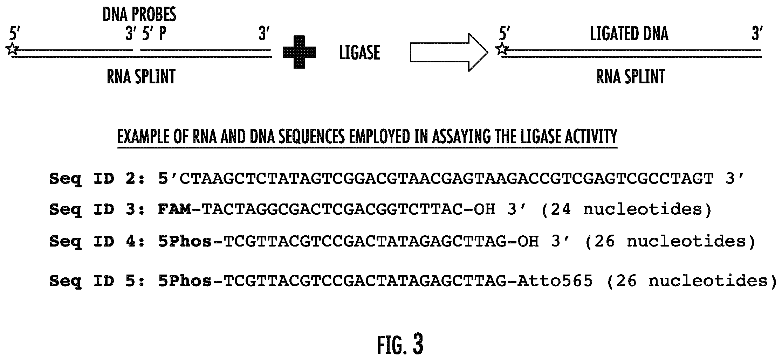

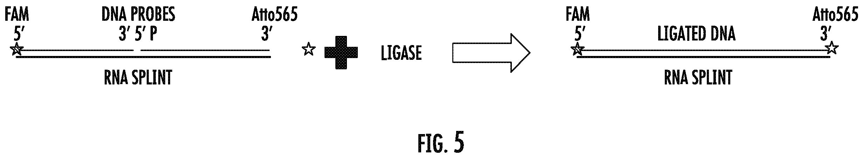

*In vitro* ligation assays were performed using a number of different sequences. Standard sequences used to assay ligation performance included a FAM labeled single stranded 24 base acceptor oligonucleotide (SEQ ID NO:3), a 5' phosphorylated single stranded 26 base donor oligonucleotide (SEQ ID NO:4) and a complementary single stranded 50 base DNA or RNA template (SEQ ID NO:2). 100nM of the pre-annealed labeled oligonucleotide complex **(****Fig. 3** and **Fig. 5****)** was ligated in reaction Buffer1 (50mM Tris-HCl pH 7.0-8.8, 10mM DTT, 0.9% synperionic acid, 0.04% wt/vol BSA, 0-10mM MgCl2, 0-1mM ATP) at 16-42°C. Reaction was assembled on ice and initiated by addition of enzyme to a final concentration ranging from 3nM - 4µM, before incubating in a thermocycler at the appropriate temperature. Reactions were quenched by heating the reaction to 95°C for 10 minutes and treated with 6U/µL of Proteinase K at 37°C for 15 minutes.

### Example 2

### Fragment Analysis by Capillary Electrophoresis

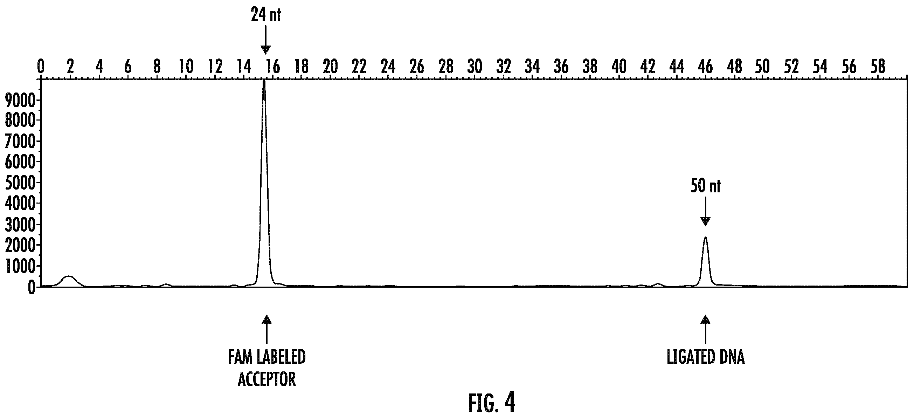

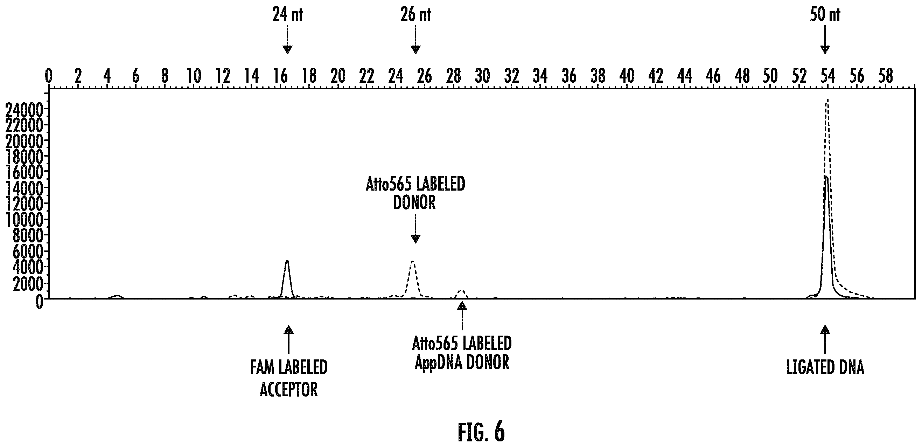

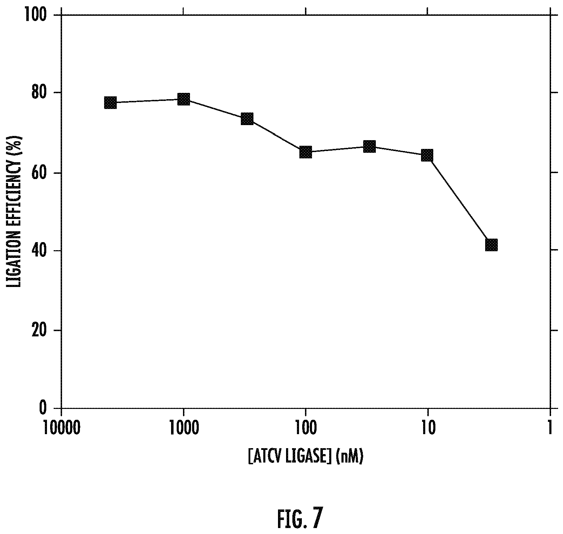

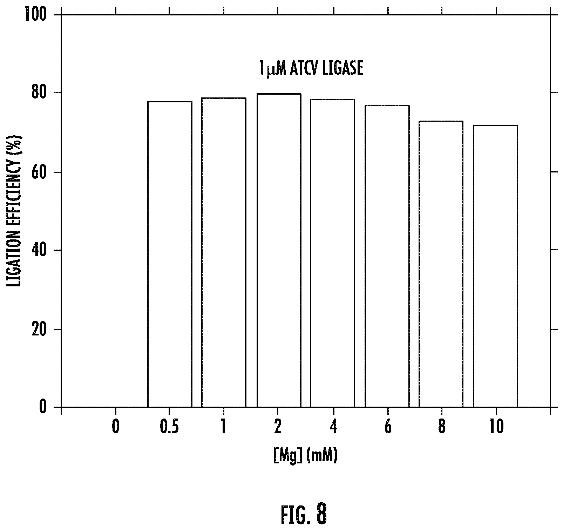

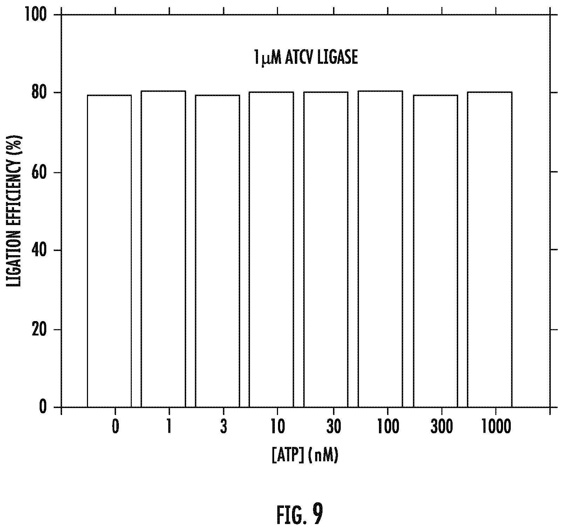

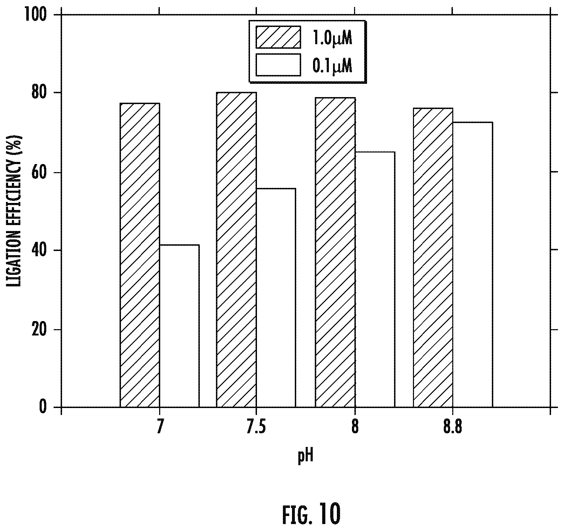

Proteinase treated reaction samples were diluted 20 fold in ddH₂O to 5nM. 1µL of the diluted sample and 1µL of GeneScan 120Liz were combined with HiDi Formamide (Thermofisher) before application to a SeqStudio Genetic Analyzer (Applied Biosystems, Carlsbad). CE data was collected using Applied Biosystems software and analyzed using PeakScanner software. Retention times and area under peak of all oligonucleotide lengths were recorded. 24 nucleotide FAM labeled oligonucleotide, FAM labeled 50 nucleotide molecules **(****Fig. 4****),** 26 nucleotide Atto dye labeled oligonucleotide and 50 nucleotide Atto dye labeled oligonucleotide **(****Fig. 6****)** were identified by coelution with synthetic standards. The fraction of each peak in the curve was determined by dividing area under individual peaks by the sum of the areas under the peaks. The efficiency of ligation is defined as the ratio of the area under the peak for the ligated product divided by the area under the curve for all peaks in the FAM channel. RNA splinted DNA ligation reactions were carried out at various enzyme concentrations and the efficiency was calculated at each enzyme concentration. Efficiency of ligation increased as the enzyme concentration was increased **(****Fig. 7****).** Efficiency of ligation as a function of magnesium concentration was characterized and measured across a wide range **(****Fig. 8****).** Ligation efficiency as a function of ATP was characterized by varying ATP concentration over a wide range of values **(****Fig. 9****).** Ligation efficiency of ATCV-1 ligase and/or the recombinant ligase composition in buffers at varying pH was tested **(****Fig. 10****).** Ligation activity and efficiency of ATCV-1 ligase and/or the recombinant ligase composition for DNA splinted DNA ligation was measured, showing that the enzyme is as efficient as PBCV-1 **(****Fig. 14A** and **Fig. 14B****).** Comparison of RNA splinted DNA ligation at 25°C and 37°C shows that the ATCV-1 ligase has higher activity at 25°C than at 37°C **(****Fig. 15A** and **Fig. 15B****).**

### Example 3

### RNA-mediated annealing, selection and ligation (RASL) assay.

Probes were designed for RASL assay such that the donor probe, phosphorylated at the 5' end, had the reverse complement of the Illumina P5 adaptor at the 3' end of a sequence that was complementary to an RNA template. The other probe was designed to include the Illumina p7 adaptor sequence 5' to the sequence complementary to the RNA template. Probes were annealed to RNA template by adding together 10nM of each DNA probe and 10nM of RNA template in ligation buffer (Buffer1 pH 8.8, T4 DNA Ligase buffer), heating to 85C for 2 minutes before cooling to 22C at a ramp rate of 0.2C/s and finally holding at 22C for 2 hours. To the annealed construct, ATCV1 ligase (to a final concentration of 100nM) or PBCV-1 Ligase (final concentration of 2.5U/µL) or T4 DNA ligase (final concentration of 40U/µL) was added in a total reaction volume of 20µL and incubated at 25C for 30 minutes before denaturing the ligase by heating to 95C for 5 minutes. The ligation reaction was diluted 100 fold in nuclease free water and 4µL of the diluted sample was assayed in a qPCR reaction using KAPA Library quantification kit and standard PCR conditions at 95C for 30 cycles.

In the absence of a template, background signal using ATCV1 ligase (Cq = 19.07) was reduced compared to PBCV-1 (18.25) or T4 DNA Ligase (18.37) **(****Fig. 11B****).** In the presence of an RNA template, signal for ATCV1 (Cq = 9.15) was improved compared to PBCV-1 (Cq = 10.66) or T4 DNA ligase (Cq = 14.55) **(****Fig. 11A****).**

### Example 4

### Application in Spatial Transcriptomics using FFPE sample

Mouse brain sections were prepared on Visium Gene Expression slides (Visium Gene Expression for FFPE, 10X Genomics, Inc.) and baked at 60C for 2 hours. Tissues were deparaffinized as follows: incubation in xylene for 10 minutes twice, incubation in 100% ethanol for 3 minutes twice, incubation at 37C for 15 minutes, incubation in 100% ethanol for 3 minutes, incubation in 96% ethanol for 3 minutes twice, incubation in 70% ethanol for 3 minutes and rinsed in MilliQ water for 20 seconds. Slides were subjected to hematoxylin and eosin staining as follows: incubation in hematoxylin for 3 minutes, rinsed in MilliQ water twice for 10-20 seconds, incubated in bluing buffer for 1 minute, rinsed in MilliQ water for 20 seconds, incubated in alcoholic eosin solution for 1 minute, and rinsed in MilliQ water for 30 seconds. Slides were coverslipped in 85% glycerol and subjected to microscope visualization using brightfield imaging. Coverslips were removed by water submersion and slide was dried at 37C for 3 minutes. Slides were placed in a plastic 10x slide cassette with gasket and tissues were washed in 100uL 0.1N HCl for 1 minute, three times. Tissues were washed in 150uL TE at pH 9.0 and then incubated with 100uL TE, pH 9.0 at 70C for 1 hour for decrosslinking. Cassette containing the slides were cooled to room temperature and TE was removed. 100uL of pre-hybridization buffer was added to the tissues and incubated at room temperature for 15 minutes.

Left handed (LHS) and right handed (RHS) probe sets designed to hybridize to a subset of gene expression targets were added to the slides and allowed to hybridize to targeted genes for 16 hours at 50C in hybridization buffer. After hybridization, tissue sections were washed in 150uL of hybridization buffer without LHS or RHS probes at 50C, three times. The reaction was cooled to room temperature, followed by probe ligation. During the ligation step, ATCV-1 Ligase was used at 1uM, 0.5uM and 0.1uM final concentrations along with PBCV-1 as a positive control at 1uM final concentration and incubated at 37C for 1 hour. Tissues were washed with 100uL post-ligation wash buffer twice at 57C for 5 min and then washed twice in 150uL of 2x SSC at room temperature. Probes hybridized to gene expression targets were released with 75uL of RNase for 30 min at 37C. Slides were treated with 75uL of tissue permeabilization reagent and incubated at 37C for 40 min, thereby releasing the ligated probes for migrating to the substrate for hybridizing with the capture domains of the capture probes on the slide. Permeabilization reagent was removed and slides were washed 3 times with 175uL 2x SSC. Probe extension was performed on the slide with 75uL of probe extension mix at 45C for 15 minutes. Slides were then washed with 100uL 2x SSC. Probes were removed from slide in presence of 40uL 0.08N KOH incubated at room temperature for 10 min and transferred to a strip tube containing 5uL 1M Tris-HCl, pH 7 for neutralization.

Samples were quantified in qPCR with specific primers designed to the removed probe design to determine the specific cycle number for SI-PCR. Samples were then subjected to SI-PCR using amplification master mix and a dual indexed TS primer plate with cycle numbers determined from qPCR. Post-PCR, samples were purified via 0.85x SPRI and eluted in 25uL elution buffer. Samples were pooled and sequenced on Illumina NovaSeq. Reaction conditions were performed in two replicates of mouse brain tissue sections. Results show decreased sensitivity in terms of panel genes and panel UMIs at similar concentrations to PBCV-1 ligase, however, both panel genes and panel UMIs are improved at 0.5uM ATCV-1 ligase and most improved at 0.1uM ATCV-1 ligase as shown in **Fig. 13A and Fig. 13B****.**

### Example 5

### Single cell RTL Application using FFPE samples

Briefly, peripheral blood mononuclear cells (PBMCs) were thawed and added to a 50mL falcon tube. 10mL of prewarmed RPMI+FBS was added slowly to the PBMCs and the cells were pelleted for 5' at 350g. Supernatant was removed and cells were washed once with 1.5mL of PBS+0.04%BSA and filtered with a 40 micron Flowmi cell strainer and transferred to a 1.5mL Eppendorf tube. PBMCs were pelleted a second time for 5' at 350g and after removing the supernatant cells were fixed with 1mL of 4% PFA diluted in PBS for 1 hour. Following fixation, PBMCs were pelleted for 5' at 850g and quenched with 100mM Tris pH8.0. Fixed PBMCs were aliquoted at ~1M cells per tube and pelleted a second time for 5' at 850g. The supernatant was removed from the tube and each 1M aliquot of PBMCs was resuspended in hybridization buffer and the working concentration of RHS [right hand side] and LHS [left hand side] DNA probes targeting the RNA transcripts of interest. Probes were hybridized overnight at 37C. After ~16 hours of hybridization, PBMCs were washed 3x 0.5mL with post-hybridization wash buffer and resuspended in 200µL of ligation buffer. Washed PBMCs were counted with the Cellaca Cell Counter and ~8000 cells were aliquoted into strip tubes in 32µL of ligation buffer. 8µL of either PBCV-1 or ATCV-1 was added to an aliquot followed by 35µL of GEM mastermix (Chromium Single Cell, 10X Genomics, Inc.). Each sample was dropletized in a 10x NextGEM chip and then GEMs were incubated at 25C for 1 hour, 55C for 45 minutes, and 80C for 20 minutes. GEMs were broken and ligation was carried out, barcoded material was preamplified for 13 cycles, cleaned up with a 1.8x SPRI, and indexed with 10x TS
plates. Libraries were sequenced on an Illumina Novaseq. Data shown in **Fig. 16** include the fraction of reads on target and mapped or "specificity" (top) which indicates the percentage of properly paired LHS and RHS probes that have been ligated as well as the median UMI counts per cell at 2500 panel reads per cell or "sensitivity" (bottom). The UMI counts represent the number of unique RTL probes that have been ligated in each cell by either PBCV-1 (right) or ATCV-1 (left).

### Example 6

### DNA Padlock Probes with ATCV-1 In Situ

Fresh frozen mouse brain tissue sections (10um) were mounted onto Superfrost slides and fixed with 3.7% formaldehyde for 5min. Samples were washed in PBS and permeabilized with 0.1M HCl for 5min. DNA or chimeric padlock probes designed to hybridize to two different gene expression targets (one specific for cortex and one for hippocampus) were added to the slides and allowed to hybridize ON at 37°C in hybridization buffer. After hybridization, tissue sections were washed two times with PBS-T at RT followed by a stringency wash for 30min at 37°C. For the ligation step, ATCV-1 Ligase was run at 0.5uM final concentrations along with T4RNA ligase 2 as a positive control at 0.5U/µl final concentration and incubated at 37°C for 2 hours. Tissues were washed twice with PBS-T followed by rolling circle amplification (RCA) performed ON with 1U/µl final concentration of Phi29. Gene-specific fluorescently labelled oligos complementary to the RCA products were then hybridized together with DAPI nuclei stain for 30 min at RT. Tissues were washed twice with PBS-T and dehydrated using an EtOH series followed by mounting with Slowfade Gold and fluorescent imaging. Data in **Fig. 17A** and **Fig. 17B** show that ATCV-1 ligation of DNA padlock probes performs similar as the positive control using chimeric probes and T4RNA ligase 2, both in terms of sensitivity and the spatial distribution of gene expression in the cortex **(****Fig. 17A****)** and hippocampus **(****Fig. 17B****).**

A schematic representation of an *in situ* platform of the present disclosure is shown in **Fig. 18****.**

### Example 7

### Plasmid Construct of ATCV-1-HU Fusion

A plasmid construct of an ATCV-1-HU (also known as LigAT-HU) recombinant ligase composition of the present disclosure was prepared.

The plasmid construct of the ATCV-1-HU recombinant ligase composition comprises a His tag and a SUMO tag upstream of the N-terminus to help with protein purification and solubility, respectively. The construct comprises an N-terminal His tag followed by a short linker (amino acids SSG) and a SUMO tag. The ATCV-1 enzyme sequence was located 3' of the SUMO tag with another linker GGSGGS (SEQ ID NO: 11) between the ATCV-1 sequence and HU sequence (at the C-terminus).

The plasmid construct comprises a nucleotide sequence of SEQ ID NO: 10, as follows:

CCCGTAGAAAAGATCAAAGGATCTTCTTGAGATCCTTTTTTTCTGCGCGTAATCTGCT
GCTTGCAAACAAAAAAACCACCGCTACCAGCGGTGGTTTGTTTGCCGGATCAAGAG
CTACCAACTCTTTTTCCGAAGGTAACTGGCTTCAGCAGAGCGCAGATACCAAATACT
GTTCTTCTAGTGTAGCCGTAGTTAGCCCACCACTTCAAGAACTCTGTAGCACCGCCT
ACATACCTCGCTCTGCTAATCCTGTTACCAGTGGCTGCTGCCAGTGGCGATAAGTCG
TGTCTTACCGGGTTGGACTCAAGACGATAGTTACCGGATAAGGCGCAGCGGTCGGG
CTGAACGGGGGGTTCGTGCACACAGCCCAGCTTGGAGCGAACGACCTACACCGAAC
TGAGATACCTACAGCGTGAGCTATGAGAAAGCGCCACGCTTCCCGAAGGGAGAAAG
GCGGACAGGTATCCGGTAAGCGGCAGGGTCGGAACAGGAGAGCGCACGAGGGAGC
TTCCAGGGGGAAACGCCTGGTATCTTTATAGTCCTGTCGGGTTTCGCCACCTCTGACT
TGAGCGTCGATTTTTGTGATGCTCGTCAGGGGGGCGGAGCCTATGGAAAAACGCCA
GCAACGCGGCCTTTTTACGGTTCCTGGCCTTTTGCTGGCCTTTTGCTCACATGTTCTTT
CCTGCGTTATCCCCTGATTCTGTGGATAACCGTATTACCGCCTTTGAGTGAGCTGATA
CCGCTCGCCGCAGCCGAACGACCGAGCGCAGCGAGTCAGTGAGCGAGGAAGCGGA

AGGCGAGAGTAGGGAACTGCCAGGCATCAAACTAAGCAGAAGGCCCCTGACGGAT
GGCCTTTTTGCGTTTCTACAAACTCTTTCTGTGTTGTAAAACGACGGCCAGTCTTAAG
CTCGGGCCCCCTGGGCGGTTCTGATAACGAGTAATCGTTAATCCGCAAATAACGTAA
AAACCCGCTTCGGCGGGTTTTTTTATGGGGGGAGTTTAGGGAAAGAGCATTTGTCAG
AATATTTAAGGGCGCCTGTCACTTTGCTTGATATATGAGAATTATTTAACCTTATAAA
TGAGAAAAAAGCAACGCACTTTAAATAAGATACGTTGCTTTTTCGATTGATGAACAC
CTATAATTAAACTATTCATCTATTATTTATGATTTTTTGTATATACAATATTTCTAGTT
TGTTAAAGAGAATTAAGAAAATAAATCTCGAAAATAATAAAGGGAAAATCAGTTTT
TGATATCAAAATTATACATGTCAACGATAATACAAAATATAATACAAACTATAAGAT
GTTATCAGTATTTATTATGCATTTAGAATAAATTTTGTGTCGCCCTTCCGCGAAATTA
ATACGACTCACTATAGGGGAATTGTGAGCGGATAACAATTCCCCTCTAGAAATAATT
TTGTTTAACTTTTTGAGACCTTAAGGAGGTAAAAAATGGGTTCTAGCCACCATCATC
ATCATCACTCTTCCGGTAGCCTGCAGGACAGCGAAGTCAATCAAGAGGCGAAGCCT
GAAGTTAAGCCAGAAGTCAAACCGGAAACGCACATTAACCTGAAAGTTTCGGATGG
CTCAAGCGAGATTTTCTTTAAGATCAAGAAAACCACGCCGTTGCGTCGCCTGATGGA
AGCGTTTGCCAAACGTCAGGGCAAAGAAATGGATTCCCTGCGCTTCCTGTATGACGG
CATCCGTATCCAGGCAGATCAAGCACCGGAAGATCTGGATATGGAAGATAACGACA
TTATTGAAGCGCACAGAGAGCAAATTGGTGGCATGGCTATTCAGAAGCCGTTACTG
GCGGCCAGCCTGAAGAAAATGTCTGTTGACAACTTGACTTTCCCGGTCTACGCAACG
CCGAAACTGGACGGTATCCGTGCGCTGAAGATTGACGGCACCCTGGTGTCGCGTAC
GTTCAAACCGATCCGCAATACTACCATCTCCAAGGTCCTGGCGAGCTTGCTGCCGGA
CGGCAGCGATGGTGAGATCTTGTCTGGTAAGACCTTTCAGGATAGCACCAGCACCGT
GATGACGACCGATGCGGGTATCGGTAGCGATACCACCTTCTTCTGGTTTGATTACGT
TAAAGACGACCCGGATAAGGGTTACCTGGACCGTATCGCTGACATGAAAACCTTCG
TCGACCAGCACCCGGAAATCCTGAAAGACAGCTGCGTGACGATCGTCCCGCTGTTCC
CGAAGAAAATTGACACGCCAGAAGAACTGCATGTGTTTGAGAAATGGTGTCTGGAC
CAGGGTTTTGAGGGCGTTATGGTGCGTACGGCCGGTGGCAAGTATAAGTTTGGTCGC
AGCACCGAGAAAGAACAAATCCTGGTTAAGATTAAACAGTTTGAAGATGATGAAGC
TGTTGTGATCGGTGTGAGCGCGCTGCAAACCAATACGAACGATAAGAAACTGAATC
AGCTGGGTGAGATGCGTCGTACTAGCCACCAAGACGGCAAAGTTGACCTGGACATG
CTGGGTGCGCTTGACGTGGACTGGAATGGTATTCGTTTCAGCATCGGTACGGGCTTT
GATAAAGACACCCGCGAAGATCTCTGGAAACAACGCGACAGCATCGTGGGTAAAGT
CGTTAAGTTCAAGTATTTCTCCCAGGGTATTAAGACCGCTCCGCGTTTTCCTGTGTTT
CTGGGTTTTCGTGACGAGAACGATATGGGTGGCAGCGGTGGCAGCATGAATAAGAC
GCAACTGATTGACGTCATTGCAGAGAAAGCCGAGTTGAGCAAGACCCAGGCGAAAG

CCGCGCTGGAGAGCACGCTGGCAGCGATTACCGAGAGCCTGAAAGAGGGCGATGCA
GTTCAACTGGTTGGCTTCGGCACCTTCAAGGTCAACCACCGTGCGGAGCGCACCGGT
CGCAACCCGCAGACCGGTAAAGAAATCAAGATTGCAGCCGCGAACGTGCCGGCGTT
CGTCAGCGGTAAGGCACTGAAAGATGCAGTAAAGTAATGAGGTTGAGGTCTCACCC
CCTAGCATAACCCCTTGGGGCCTCTAAACGGGTCTTGAGGGGTTTTTTGCCCCTGAG
ACGCGTCAATCGAGTTCGTACCTAAGGGCGACACCCCCTAATTAGCCCGGGCGAAA
GGCCCAGTCTTTCGACTGAGCCTTTCGTTTTATTTGATGCCTGGCAGTTCCCTACTCT
CGCATGGGGAGTCCCCACACTACCATCGGCGCTACGGCGTTTCACTTCTGAGTTCGG
CATGGGGTCAGGTGGGACCACCGCGCTACTGCCGCCAGGCAAACAAGGGGTGTTAT
GAGCCATATTCAGGTATAAATGGGCTCGCGATAATGTTCAGAATTGGTTAATTGGTT
GTAACACTGACCCCTATTTGTTTATTTTTCTAAATACATTCAAATATGTATCCGCTCA
TGAGACAATAACCCTGATAAATGCTTCAATAATATTGAAAAAGGAAGAATATGAGC
CATATTCAACGGGAAACGTCGAGGCCGCGATTAAATTCCAACATGGATGCTGATTTA
TATGGGTATAAATGGGCTCGCGATAATGTCGGGCAATCAGGTGCGACAATCTATCGC
TTGTATGGGAAGCCCGATGCGCCAGAGTTGTTTCTGAAACATGGCAAAGGTAGCGTT
GCCAATGATGTTACAGATGAGATGGTCAGACTAAACTGGCTGACGGAATTTATGCC
ACTTCCGACCATCAAGCATTTTATCCGTACTCCTGATGATGCATGGTTACTCACCACT
GCGATCCCCGGAAAAACAGCGTTCCAGGTATTAGAAGAATATCCTGATTCAGGTGA
AAATATTGTTGATGCGCTGGCAGTGTTCCTGCGCCGGTTGCACTCGATTCCTGTTTGT
AATTGTCCTTTTAACAGCGATCGCGTATTTCGCCTCGCTCAGGCGCAATCACGAATG
AATAACGGTTTGGTTGATGCGAGTGATTTTGATGACGAGCGTAATGGCTGGCCTGTT
GAACAAGTCTGGAAAGAAATGCATAAACTTTTGCCATTCTCACCGGATTCAGTCGTC
ACTCATGGTGATTTCTCACTTGATAACCTTATTTTTGACGAGGGGAAATTAATAGGTT
GTATTGATGTTGGACGAGTCGGAATCGCAGACCGATACCAGGATCTTGCCATCCTAT
GGAACTGCCTCGGTGAGTTTTCTCCTTCATTACAGAAACGGCTTTTTCAAAAATATG
GTATTGATAATCCTGATATGAATAAATTGCAGTTTCATTTGATGCTCGATGAGTTTTT
CTAAGCGGCGCGCCATCGAATGGCGCAAAACCTTTCGCGGTATGGCATGATAGCGC
CCGGAAGAGAGTCAATTCAGGGTGGTGAATATGAAACCAGTAACGTTATACGATGT
CGCAGAGTATGCCGGTGTCTCTTATCAGACCGTTTCCCGCGTGGTGAACCAGGCCAG
CCACGTTTCTGCGAAAACGCGGGAAAAAGTGGAAGCGGCGATGGCGGAGCTGAATT
ACATTCCCAACCGCGTGGCACAACAACTGGCGGGCAAACAGTCGTTGCTGATTGGC
GTTGCCACCTCCAGTCTGGCCCTGCACGCGCCGTCGCAAATTGTCGCGGCGATTAAA
TCTCGCGCCGATCAACTGGGTGCCAGCGTGGTGGTGTCGATGGTAGAACGAAGCGG
CGTCGAAGCCTGTAAAGCGGCGGTGCACAATCTTCTCGCGCAACGCGTCAGTGGGC
TGATCATTAACTATCCGCTGGATGACCAGGATGCCATTGCTGTGGAAGCTGCCTGCA

CTAATGTTCCGGCGTTATTTCTTGATGTCTCTGACCAGACACCCATCAACAGTATTAT
TTTCTCCCATGAGGACGGTACGCGACTGGGCGTGGAGCATCTGGTCGCATTGGGTCA
CCAGCAAATCGCGCTGTTAGCGGGCCCATTAAGTTCTGTCTCGGCGCGTCTGCGTCT
GGCTGGCTGGCATAAATATCTCACTCGCAATCAAATTCAGCCGATAGCGGAACGGG
AAGGCGACTGGAGTGCCATGTCCGGTTTTCAACAAACCATGCAAATGCTGAATGAG
GGCATCGTTCCCACTGCGATGCTGGTTGCCAACGATCAGATGGCGCTGGGCGCAATG
CGCGCCATTACCGAGTCCGGGCTGCGCGTTGGTGCGGATATCTCGGTAGTGGGATAC
GACGATACCGAAGATAGCTCATGTTATATCCCGCCGTTAACCACCATCAAACAGGAT
TTTCGCCTGCTGGGGCAAACCAGCGTGGACCGCTTGCTGCAACTCTCTCAGGGCCAG
GCGGTGAAGGGCAATCAGCTGTTGCCAGTCTCACTGGTGAAAAGAAAAACCACCCT
GGCGCCCAATACGCAAACCGCCTCTCCCCGCGCGTTGGCCGATTCATTAATGCAGCT
GGCACGACAGGTTTCCCGACTGGAAAGCGGGCAGTGACTCATGACCAAAATCCCTT
AACGTGAGTTACGCGCGCGTCGTTCCACTGAGCGTCAGAC (SEQ ID NO:10)

In reference to the nucleotide sequence of SEQ ID NO:10 (above), the His tag corresponds to bases 1459-1488, the SUMO tag corresponds to bases 1498-1794, ATCV-1 corresponds to bases 1795-2694, and HU corresponds to bases 2712-2982.

### Example 8

### Protein Expression and Purification of ATCV-1-HU

The plasmid encoding the enzyme of interest was transformed into *E. coli* expression strain BL21 DE3 and plated on LB agar plates with kanamycin as the antibiotic selection agent. A starter culture of LB+kanamycin (50ug/mL final concentration) was inoculated with a single colony from the LB agar plate overnight at 37C. A 1L LB culture supplemented with kanamycin was inoculated with 5mL of the starter culture and grown with shaking at 37C until the OD reached 0.8. Expression was induced with 0.5mM IPTG at 18C overnight. The cells were harvested by centrifugation and resuspended in lysis buffer (50mM Tris pH 8, 500mM NaCl, 10% glycerol) and lysed by sonication. The lysate was clarified by centrifugation and the soluble protein in the supernatant was collected. The lysate was applied to a nickel column equilibrated with Buffer A (50mM Tris pH 8, 500mM NaCl, 10mM Imidazole) to bind the His tagged proteins using an AKTA Pure 25 instrument. Non-specifically bound proteins were washed with Buffer A containing 40mM Imidazole and eluted by a gradient from 40 to 500mM Imidazole. Nickel column elution fractions with protein of interest as observed on a gel were collected and buffer exchanged into 50mM Tris pH 8, 100mM NaCl, 1mM DTT. Ulp1 protease was added to the protein at a mass ratio of 1:1000 Ulp1:SUMO-tagged-protein and incubated overnight. The cleaved protein was passed over a Nickel column and flow through containing the cleaved protein of interest was collected and further cleaned using a Heparin affinity column. For the Heparin step the column was equilibrated with Buffer C (50mM Tris pH 8, 100mM NaCl) and the flow through from the second nickel column was applied to it. The ATCV-1-HU enzyme was eluted using a gradient from 100mM NaCl to 1M NaCl. Peak fractions as observed on an SDS PAGE gel were collected and exchanged into storage buffer (10mM Tris HCl pH 7.5, 100mM KCl, 1mM DTT, 0.5mM EDTA) for storage at -20C until use in experiments.

### Example 9

### Characterization of ATCV-1-HU

Various experiments were conducted to characterize the ATCV-1-HU recombinant ligase composition of the present disclosure, including: ligase activity and efficiency assay **(****Fig. 19****);** enzyme titration **(****Fig.20****);** efficiency comparison using molar concentration titration **(****Fig. 21****);** efficiency comparison using unit activity titration **(****Fig. 22****);** ligation efficiency comparison of ATCV-1, ATCV-1-HU, and PBCV-1 in the presence of high salt **(****Fig. 23****);** ligation of RNA templated DNA probes with 3' ribonucleotide base **(****Fig. 24****);** and *in situ* mouse brain ligation comparison of ATCV-1 and ATCV-1-HU (DNA templated DNA ligation) **(****Fig. 25****).**

### Example 10

### Comparison of ATCV-1 and ATCV-1-HU

Experiments were conducted to compare ATCV-1 and ATCV-1-HU for qualitative performance and sensitivity. Results indicate that ATCV-1-HU outperformed ATCV-1. Qualitative results comparing ATCV-1 and ATCV-1- HU demonstrated that ATCV-1-HU appeared to have outperformed ATCV-1 in generating an increased number of ligation events by feature on a spatial array. Sensitivity results comparing ATCV-1 and ATCV-1-HU are shown in **Fig. 26****,** demonstrating that sensitivity for ATCV-1-HU was several fold higher that the non-fusion ATCV-1 enzyme in mouse cortex. Sensitivity signal results comparing ATCV-1 and ATCV-1-HU in mouse cortex are shown in **Fig. 27****,** which shows that, regardless of enzyme used, the signal intensity was comparable for detecting the Satb2 gene.

### Example 11

### Single Cell Analysis in PBMC and Jurkat Cells

Cryopreserved cells, either peripheral blood mononuclear cells (PBMCs) or cryopreserved Jurkat cells, were thawed, combined with 10 mL of RPMI+FBS that had been prewarmed at 37C, and pelleted by centrifugation for 5 min at 350g. Supernatant was removed and cells were washed once with 1.5 mL of PBS + 0.04% BSA and transferred to a 1.5 mL Eppendorf tube. Cells were pelleted a second time for 5 min at 350g and after removing the supernatant, cells were fixed with 1 mL of Fixation and Permeabilization buffer containing 4% PFA for 1 hour at room-temperature. Following fixation, cells were pelleted for 5 min at 850g and quenched with Quenching solution. Fixed cells were aliquoted at ~1 million cells per tube and pelleted a second time for 5 min at 850g. The supernatant was removed from the tube and each aliquot (approx. 1 million cells/aliquot) was resuspended in hybridization buffer and the working concentration of RHS [right hand side] and LHS [left hand side] DNA probes targeting the RNA transcripts of interest. Probes were hybridized overnight at 37C or 42C. After ~16 hours of hybridization, cells were washed either two or three times with post-hybridization wash buffer and resuspended in Cell Resuspension buffer and filtered with a 40 micron Flowmi cell strainer, or a 30 micron CellTrics cell strainer. Washed cells were counted with the Cellaca Cell Counter and then between 4000-5000 cells were aliquoted into strip tubes in cell resuspension buffer, to which ligase enzyme (either PBCV-1, ATCV-1, or ATCV-1-HU) was added followed by 35 µl of GEM mastermix, for a total of 75 µl. Each sample was dropletized in a 10x NextGEM chip and then GEMs were incubated at 25C for 1 hour, 55C or 60C for 45 min, and 80C for 20 min. GEMs were broken, ligations performed and barcoded material was PCR-amplified for 8 cycles, cleaned up with a 1.8X SPRI, and indexed with 10x TS plates. Libraries were sequenced on an Illumina Novaseq. Results from one such experiment in Jurkat cells are summarized in **Fig. 28****.** The results shown in **Fig. 28** indicate ATCV-1 and PBCV-1 enzymes perform comparably in the scRTL assay in terms of both data quality and sensitivity. Results from one such experiment in Jurkat cells are summarized in **Fig. 29** indicate ATCV-1-HU and PBCV-1 enzymes perform comparably in the scRTL assay in terms of both data quality and sensitivity. The close overlay of results obtained with ATCV-1-HU and PBCV-1 as shown in **Fig. 30** indicate the ligases performed comparably in this assay. Similar results were obtained with PBMC cells as shown in **Fig. 31****.**

### Example 12

### Single Cell RTL Evaluation of Ligases in Human Spleen Cells

Flash-frozen human spleen tissue was cut into small pieces, and fixed with 1 mL of Fixation and Permeabilization buffer containing 4% PFA for 1 hour at room-temperature. Following fixation, fixed tissue was pelleted for 5 min at 850g and quenched with Quenching solution. Fixed tissue was then enzymatically and mechanically dissociated. After dissociation, dissociated material was filtered using a 70 micron cell strainer, and cells from the filtered solution were pelleted for 5 min at 850g. Fixed spleen cells were resuspended in Quenching solution, and then filtered through a 40 micron Flowmi cell strainer. Fixed cells were aliquoted at ~1 million cells per tube and pelleted a second time for 5 min at 850g. The supernatant was removed from the tube and each 1 million aliquot of cells was resuspended in hybridization buffer and the working concentration of RHS [right hand side] and LHS [left hand side] DNA probes targeting the RNA transcripts of interest. Probes were hybridized overnight at 42C. After ~16 hours of hybridization, cells were washed three times with post-hybridization wash buffer and resuspended in Cell Resuspension buffer and filtered with a 30 micron CellTrics cell strainer. Washed cells were counted with the Cellaca Cell Counter and then between 4000 cells were aliquoted into strip tubes in cell resuspension buffer, to which ligase enzyme (either PBCV-1, ATCV-1, or ATCV-HU) was added followed by 35 µl of GEM mastermix, for a total of 75 µl. Each sample was dropletized in a 10x NextGEM chip and then GEMs were incubated at 25C for 1 hour, 60C for 45 min, and 80C for 20 min. GEMs were broken and the ligated and barcoded material was PCR-amplified for 8 cycles, cleaned up with a 1.8X SPRI, and then indexed with 10x TS plates. Libraries were sequenced on an Illumina Novaseq.

Results from one such experiment with human spleen cells are summarized in **Fig. 33****.** The overlay of results obtained with ATCV-1, ATCV_1-HU and PBCV-1 indicate the ligases are functional in this assay. ATCV-1 and ATCV-1-HU yield similar results in the context of this assay.

### Example 13

### Ability of ATCV-1 and ATCV-1-HU to ligate mismatches, or SNPs

Experiments were undertaken to determine with the ATCV-1 or ATCV-1-HU fusion ligase would be able to discern mismatches and the end, or near the end of one of both of the LHS or RHS oligonucleotides.

The oligonucleotides used in an experiment are found in **FIG. 34A****.** Along with the control probe partners (CTL), six different probe pairs were evaluated for the ability of ATCV-1 and ATCV-2-HU to discern and ligate a probe with a SNP to a second probe. For example, the probes pairs labeled 3PMM1, 3PMM2, and 3PPM1 each have a SNP the 1^{st}, 2^{nd}, or 3^{rd} position at the 3' end of the left hand probe (SNP underlined). Conversely, 5PMM1, 5PMM2 and 5PMM3 each have a SNP at the 1^{st}, 2^{nd} or 3^{rd} position from the end of the 5' phosphorylated 5' end of the right hand probe (SNP underlined). Ligation reactions were carried out as found in the art.

Overall, the results show that the ATCV-1-HU was better at discerning a SNP resulting in lesser ligation efficiency as evidenced by ligation efficiency for SNPS that were found at the first position on the 3' end of the left hand probe and for those SNPs found at either the first or second positions at the 5' end of the right have probe **(****FIG. 34B****).** It was further demonstrated that discrimination of mismatched nucleotides was enhanced in the presence of high salt, at least for ATCV-1-HU fusion enzyme.

Another set of experiments explored whether ACTV-1-HU showed a base bias for discrimination. **FIG. 35A** shows the template oligonucleotide, control probe pair (CTL), and the three SNP probe pair sets 5PMM1_A, 5PMM1_C and 5PMM1_G (SNP underlined). As shown in **FIG. 35B****,** as previously demonstrated ACTV-1-HU discrimination was enhanced by high salt. Further, there was a slight G bias discrimination.

Notwithstanding the foregoing description or the appended claims, the disclosure set forth herein is also defined by the following numbered clauses, which may be beneficial alone or in combination, with one or more other causes or embodiments. Each of these individually numbered clauses may be used or combined with any of the preceding or following clauses. Thus, these clauses are intended to provide support for all such combinations and is not necessarily limited to specific combinations explicitly provided below:
1. A recombinant ligase composition comprising a recombinantly-produced ligase isolated *from Acanthocystis turfacea* chlorella virus 1 (ATCV-1) fused to at least one polynucleotide-binding polypeptide.
2. The recombinant ligase composition of clause 1, wherein the ATCV-1 ligase fused to at least one polynucleotide binding polypeptide comprises an amino acid ligase sequence that is at least 90% identical to SEQ ID NO: 1, at least 95% identical to SEQ ID NO:1, or at least 99% identical to SEQ ID NO: 1.
3. The recombinant ligase composition of clause 1, wherein the at least one polynucleotide-binding polypeptide is a DNA-binding polypeptide or an RNA-binding polypeptide.
4. The recombinant ligase composition of clause 1, wherein the polynucleotide-binding polypeptide is a HU protein or a functional variant or functional fragment thereof.
5. The recombinant ligase composition of clause 4, wherein the HU protein has an amino acid sequence that is at least 90% identical to SEQ ID NO:7, at least 95% identical to SEQ ID NO:2, or at least 99% identical to SEQ ID NO:7.
6. The recombinant ligase composition of clause 1, wherein the recombinantly-produced ligase has nucleotide sequence that is at least 90% identical to SEQ ID NO:8, at least 95% identical to SEQ ID NO:8, or at least 99% identical to SEQ ID NO:8.
7. The recombinant ligase composition of clause 1, wherein the recombinantly-produced ligase has an amino acid sequence that is at least 90% identical to SEQ ID NO:9, at least 95% identical to SEQ ID NO:9, or at least 99% identical to SEQ ID NO:9.
8. The recombinant ligase composition of clause 1 further comprising a label.
9. The recombinant ligase composition of clause 8, wherein the label is attached to the recombinantly-produced ligase.
10. The recombinant ligase composition of clause 8, wherein the label is a detectable label.
11. The recombinant ligase composition of clause 10, wherein the detectable label is a fluorescent label or an isotopic label.
12. The recombinant ligase composition of clause 8, wherein the label is a purification label.
13. The recombinant ligase composition of clause 12, wherein the purification label is a tag selected from the group consisting of a poly-histidine tag, a streptavidin tag, a biotin tag, and a peptide tag.
14. A method for ligating double-stranded DNA or single-stranded DNA together using the recombinant ligase composition of any one of clauses 1-13 to complete the ligation.
15. A method for ligating DNA polynucleotide probes together, said method comprising: (a) providing a nucleic acid template; (b) hybridizing at least two DNA polynucleotide probes to the nucleic acid template; and (c) ligating the at least two DNA polynucleotide probes to one another using the recombinant ligase composition of any one of clauses 1-13.
16. The method of clause 15, wherein the nucleic acid template comprises a splice junction.
17. The method of clause 16, wherein at least a portion of the DNA polynucleotide probes have complementary regions at the splice junction to the nucleic acid template.
18. The method of clause 15, wherein the nucleic acid template is an RNA template.
19. The method of clause 15, wherein the two DNA polynucleotide probes are permitted to ligate to form a single polynucleotide.
20. The method of clause 15, wherein the ligating is performed in a buffer containing at least 1 µM - 1.5 mM ATP.
21. The method of clause 15, wherein the ligating step further comprises incubating for a time period effective to achieve at least 70%- 90% ligation of the DNA polynucleotide probes.
22. A method for detecting RNA sequences, said method comprising: (a) annealing polynucleotides having regions that are complementary at a ligation junction to an RNA template; (b) ligating the polynucleotides using the recombinant ligase composition of any one of clauses 1-13; (c) amplifying the ligation product; and (d) detecting and optionally quantifying the amplification product.
23. The method of clause 22, wherein the RNA template is a microRNA.
24. A method for identifying the presence or absence of one or more nucleic acids in a biological sample, said method comprising: (a) providing the biological sample on an array comprising a plurality of capture probes; (b) contacting a first probe and a second probe with the biological sample, wherein the first probe and the second probe each comprise one or more sequences that are substantially complementary to sequences of the analyte, and wherein the second probe comprises a capture probe capture domain; (c) hybridizing the first probe and the second probe to the analyte; and (d) generating a ligation product by ligating the first probe and the second probe with the recombinant ligase composition of any one of clauses 1-13.
25. The method of clause 24, wherein a capture probe of the plurality of capture probes comprises: (i) a spatial barcode and (ii) a capture domain.
26. The method of clause 25, further comprising (e) releasing the ligated product from the analyte.
27. The method of clause 26, wherein the ligation product further comprises a capture domain complementary to the capture domain of the capture probe and further comprising (f) hybridizing the ligation product to the capture domain.
28. The method of clause 27, further comprising (g) determining (i) all or a part of the sequence of the ligation product bound to the capture domain, or a complement thereof, and (ii) all or a part of the sequence of the spatial barcode, or a complement thereof, and using the determined sequence of (i) and (ii) to identify the location of the nucleic acid in the biological sample.
29. A method for single-cell analysis of one or more nucleic acids in a biological sample, said method comprising: (a) providing (i) a sample comprising a cell, wherein said cell comprises a nucleic acid molecule that comprises a first target region and a second target region, wherein said first target region and said second target region are disposed on a same strand of said nucleic acid molecule, (ii) a first probe comprising a first probe sequence that is complementary to said first target region of said nucleic acid molecule, and (iii) a second probe comprising a second probe sequence that is complementary to said second target region of said nucleic acid molecule; (b) subjecting said sample to conditions sufficient to hybridize said first probe to said first target region of said nucleic acid molecule and said second probe to said second target region of said nucleic acid molecule yield a probe-associated nucleic acid molecule in the cell; (c) partitioning the cell from step (b) into a partition, wherein the partition comprises a nucleic acid barcode molecule that comprises a partition barcode sequence; and (d) in the partition, ligating the first probe to the second probe with the recombinant ligase composition of any one of clauses 1-13 and attaching the nucleic acid barcode molecule to the first or the second probe to yield a barcoded probe-linked nucleic acid molecule.
30. A method for single-cell analysis of one or more analytes in a biological sample, said method comprising: (a) providing (i) a sample comprising a cell, wherein said cell comprises a nucleic acid molecule that comprises a first target region and a second target region, wherein said first target region and said second target region are disposed on a same strand of said nucleic acid molecule, (ii) a first probe comprising a first probe sequence that is complementary to said first target region of said nucleic acid molecule, and (iii) a second probe comprising a second probe sequence that is complementary to said second target region of said nucleic acid molecule; (b) subjecting said sample to conditions sufficient to hybridize said first probe to said first target region of said nucleic acid molecule and said second probe to said second target region of said nucleic acid molecule and ligate the first probe to the second probe with the recombinant ligase composition of any one of clauses 1-13 to yield a probe-linked nucleic acid molecule in the cell; (c) partitioning the cell from step (b) into a partition, wherein the partition comprises a nucleic acid barcode molecule that comprises a partition barcode sequence; and (d) in the partition, attaching the nucleic acid barcode molecule to the first or the second probe to yield a barcoded probe-linked nucleic acid molecule.
31. A method for *in situ* analysis of one or more nucleic acids in a biological sample, said method comprising: (a) contacting a biological sample comprising a target nucleic acid with a target padlock probe comprising targeting regions capable of hybridization to the target nucleic acid, wherein the target padlock probe is hybridized to the target nucleic acid; (b) generating a circular template by ligating the target padlock probe with the recombinant ligase composition of any one of clauses 1-13; and (c) amplifying the circular template to form an amplification product.
32. The method of clause 31, wherein the padlock probe further comprises one or more target barcode regions.
33. The method of clause 32, wherein the circular template comprises the one or more target barcode regions.
34. The method of clause 33, wherein the amplification product comprises the one or more target barcode regions or complementary sequences thereof.
35. The method of clause 34, further comprising (d) contacting the amplification product with a secondary probe comprising (1) a barcode-binding region capable of hybridization to the target barcode region or a complementary sequence thereof directly or indirectly, and (2) a detection barcode region, wherein the secondary probe is hybridized to the amplification product.
36. The method of clause 35, further comprising (e) contacting the secondary probe with a detection probe comprising (i) a barcode-binding region capable of hybridization to the detection barcode region, and (2) a detectable label, wherein the detection probe is hybridized to the secondary probe.
37. The method of clause 36, further comprising (f) detecting the detectable label, thereby providing a detectable signal for analysis of the target nucleic acid in the sample.
38. A composition, comprising the recombinant ligase composition of any one of clauses 1-13 and at least one polynucleotide having a length of at least 8 nucleotides.
39. The composition of clause 38, wherein the at least one polynucleotide is a single stranded RNA.
40. The composition of clause 38, wherein the at least one polynucleotide is a double stranded DNA, a partially double stranded DNA, a single stranded DNA, or an RNA:DNA duplex.
41. The composition of clause 38, wherein the at least one polynucleotide is a complex comprising a single stranded RNA having one or two DNA polynucleotides annealed to the single stranded RNA.
42. The composition of clause 38, wherein the recombinant ligase composition is immobilized on a bead.
43. The composition of clause 38, wherein the ligase is thermostable.
44. The composition of clause 43, wherein the ligase is thermostable at a temperature of at least 16°C, at least 25°C, at least 37°C, or at least 50°C.
45. The composition of clause 38, wherein the recombinant ligase composition has a ligation efficiency of between about 65-75% at a salt concentration of between about 400-500 mM NaCl.
46. The composition of clause 38, wherein the recombinant ligase composition has a ligation efficiency of between about 75-85% at a salt concentration of between about 100-300 mM NaCl.
47. The composition of clause 38, wherein the recombinant ligase composition has a ligation efficiency of between about 85-99% at a salt concentration of between about 0-100 mM NaCl.
48. The composition of clause 38, wherein the recombinant ligase composition has a ligation efficiency of between about 65-75% at a ligase concentration of about 0.05 U/microliter.
49. The composition of clause 38, wherein the recombinant ligase composition has a ligation efficiency of between about 85-90% at a ligase concentration of between about 0.1 and 1.0 U/microliter.
50. The composition of clause 38 further comprising a buffer.
51. The composition of clause 50, wherein the buffer comprises 1 µM - 1.5 mM ATP.
52. A method for ligating at least two polynucleotides, said method comprising a) obtaining RNA from a sample, b) hybridizing at least two DNA polynucleotides to the RNA, c) ligating the at least two DNA polynucleotides using a ligase that is at least 90% identical to SEQ ID NO:1 and d) detecting the product of step (c).
53. The method of clause 52 wherein the ligase is further fused to an amino acid sequence that is at least 90% identical to SEQ ID NO:7.
54. The method of clauses 52 and 53, wherein the RNA is mRNA.
55. The methods of clauses 52 to 54, wherein the ligase of SEQ ID NO:1 is *from Acanthocystis turfacea* chlorella virus 1.
56. The methods of any of clauses 52 to 55 wherein the detecting comprises identifying the presence or absence of a polymorphism.
57. The method of clause 56 wherein the polymorphism is a base pair mismatch, an insertion, a deletion or a splice variant in the target RNA.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

### EMBODIMENTS OF THE INVENTION

1. A recombinant ligase composition, comprising:
   a recombinantly-produced ligase isolated from *Acanthocystis turfacea* chlorella virus 1 (ATCV-1) fused to at least one polynucleotide-binding polypeptide.
2. The recombinant ligase composition of embodiment 1, wherein the ATCV-1 ligase fused to at least one polynucleotide binding polypeptide comprises a ligase amino acid sequence that is at least 90% identical to SEQ ID NO: 1, at least 95% identical to SEQ ID NO: 1, or at least 99% identical to SEQ ID NO: 1.
3. The recombinant ligase composition of embodiment 1, wherein the at least one polynucleotide-binding polypeptide is a DNA-binding polypeptide or an RNA-binding polypeptide.
4. The recombinant ligase composition of embodiment 1, wherein the polynucleotide-binding polypeptide is a HU protein or a functional variant or functional fragment thereof.
5. The recombinant ligase composition of embodiment 4, wherein the HU protein has an amino acid sequence that is at least 90% identical to SEQ ID NO:7, at least 95% identical to SEQ ID NO:2, or at least 99% identical to SEQ ID NO:7.
6. The recombinant ligase composition of embodiment 1, wherein the recombinantly-produced ligase has nucleotide sequence that is at least 90% identical to SEQ ID NO:8, at least 95% identical to SEQ ID NO:8, or at least 99% identical to SEQ ID NO:8.
7. The recombinant ligase composition of embodiment 1, wherein the recombinantly-produced ligase has an amino acid sequence that is at least 90% identical to SEQ ID NO:9, at least 95% identical to SEQ ID NO:9, or at least 99% identical to SEQ ID NO:9.
8. The recombinant ligase composition of embodiment 1 further comprising a label.
9. The recombinant ligase composition of embodiment 8, wherein the label is attached to the recombinantly-produced ligase.
10. The recombinant ligase composition of embodiment 8, wherein the label is a detectable label.
11. The recombinant ligase composition of embodiment 10, wherein the detectable label is a fluorescent label or an isotopic label.
12. The recombinant ligase composition of embodiment 8, wherein the label is a purification label.
13. The recombinant ligase composition of embodiment 12, wherein the purification label is a tag selected from the group consisting of a poly-histidine tag, a streptavidin tag, a biotin tag, and a peptide tag.
14. A method for ligating double-stranded DNA or single-stranded DNA together using the recombinant ligase composition of any one of embodiments 1-13 to complete the ligation.
15. A method for ligating DNA polynucleotide probes together, said method comprising:
   (a) providing a nucleic acid template;
   (b) hybridizing at least two DNA polynucleotide probes to the nucleic acid template; and
   (c) ligating the at least two DNA polynucleotide probes to one another using the recombinant ligase composition of any one of embodiments 1-13.
16. The method of embodiment 15, wherein the nucleic acid template comprises a splice junction.
17. The method of embodiment 16, wherein at least a portion of the DNA polynucleotide probes have complementary regions at the splice junction to the nucleic acid template.
18. The method of embodiment 15, wherein the nucleic acid template is an RNA template.
19. The method of embodiment 15, wherein the two DNA polynucleotide probes are permitted to ligate to form a single polynucleotide.
20. The method of embodiment 15, wherein the ligating is performed in a buffer containing at least 1 µM - 1.5 mM ATP.
21. The method of embodiment 15, wherein the ligating step further comprises incubating for a time period effective to achieve at least 70%- 90% ligation of the DNA polynucleotide probes.
22. A method for detecting RNA sequences, said method comprising:
   (a) annealing polynucleotides having regions that are complementary at a ligation junction to an RNA template;
   (b) ligating the polynucleotides using the recombinant ligase composition of any one of embodiments 1-13;
   (c) amplifying the ligation product; and
   (d) detecting and optionally quantifying the amplification product.
23. The method of embodiment 22, wherein the RNA template is a microRNA.
24. A composition, comprising:
   the recombinant ligase composition of any one of embodiments 1-13; and
   at least one polynucleotide having a length of at least 8 nucleotides.
25. The composition of embodiment 34, wherein the at least one polynucleotide is a single stranded RNA.
26. The composition of embodiment 24, wherein the at least one polynucleotide is a double stranded DNA, a partially double stranded DNA, a single stranded DNA, or an RNA:DNA duplex.
27. The composition of embodiment 24, wherein the at least one polynucleotide is a complex comprising a single stranded RNA having one or two DNA polynucleotides annealed to the single stranded RNA.
28. The composition of embodiment 24, wherein the recombinant ligase composition is immobilized on a bead.
29. The composition of embodiment 24, wherein the ligase is thermostable.
30. The composition of embodiment 29, wherein the ligase is thermostable at a temperature of at least 16°C, at least 25°C, at least 37°C, or at least 50°C.
31. The composition of embodiment 24, wherein the recombinant ligase composition has a ligation efficiency of between about 65-75% at a salt concentration of between about 400-500 mM NaCl.
32. The composition of embodiment 24, wherein the recombinant ligase composition has a ligation efficiency of between about 75-85% at a salt concentration of between about 100-300 mM NaCl.
33. The composition of embodiment 24, wherein the recombinant ligase composition has a ligation efficiency of between about 85-99% at a salt concentration of between about 0-100 mM NaCl.
34. The composition of embodiment 24, wherein the recombinant ligase composition has a ligation efficiency of between about 65-75% at a ligase concentration of about 0.05 U/microliter.
35. The composition of embodiment 24, wherein the recombinant ligase composition has a ligation efficiency of between about 85-90% at a ligase concentration of between about 0.1 and 1.0 U/microliter.
36. The composition of embodiment 24 further comprising a buffer.
37. The composition of embodiment 36, wherein the buffer comprises 1 µM - 1.5 mM ATP.
38. A method for ligating at least two polynucleotides, comprising:
   a) obtaining RNA from a sample,
   b) hybridizing at least two DNA polynucleotides to the RNA,
   c) ligating the at least two DNA polynucleotides using a ligase that is at least 90% identical to SEQ ID NO: 1 wherein said ligase is fused to an amino acid sequence that is at least 90% identical to SEQ ID NO: 7, and
   d) detecting the product of step (c).
39. The method of embodiment 38, wherein the RNA is mRNA.
40. The method of embodiments 38, wherein the ligase of SEQ ID NO: 1 is from *Acanthocystis turfacea* chlorella virus 1 and the fused amino acid sequence is a HU DNA binding protein.
41. The methods of embodiments 38 to 40, wherein the detecting comprising identifying the presence or absence of a polymorphism.
42. The method of embodiment 41, wherein the polymorphism is a base pair mismatch, an insertion, a deletion, or a splice variant in the target RNA.

## Claims

1. A composition, comprising: a recombinantly-produced ligase isolated from *Acanthocystis turfacea* chlorella virus 1 (ATCV-1) fused to at least one polynucleotide-binding polypeptide;
wherein the ATCV-1 ligase comprises an amino acid sequence that is at least 90% identical to SEQ ID NO: 1; and wherein the polynucleotide-binding polypeptide comprises an amino acid sequence that is at least 90% identical to SEQ ID NO: 7; and at least one polynucleotide having a length of at least 8 nucleotides.

2. The composition of claim 1, wherein the at least one polynucleotide is:
(a) a single stranded RNA;
(b) a double stranded DNA, a partially double stranded DNA, a single stranded DNA, or an RNA:DNA duplex; or
(c) a complex comprising a single stranded RNA having one or two DNA polynucleotides annealed to the single stranded RNA.

3. The composition of claim 1 or claim 2, wherein the at least one polynucleotide comprises one or more detectable labels.

4. The composition of any one of claims 1 to 3, wherein the recombinant ligase composition is immobilized on a bead.

5. The composition of claim 1, wherein the ligase is thermostable.

6. The composition of claim 5, wherein the ligase is thermostable at a temperature of at least 16°C, at least 25°C, at least 37°C, or at least 50°C.

7. The composition of claim 1, wherein the recombinant ligase composition has:
(a) a ligation efficiency of between about 65-75% at a salt concentration of between about 400-500 mM NaCl;
(b) a ligation efficiency of between about 75-85% at a salt concentration of between about 100-300 mM NaCl;
(c) a ligation efficiency of between about 85-99% at a salt concentration of between about 0-100 mM NaCl;
(d) a ligation efficiency of between about 65-75% at a ligase concentration of about 0.05 U/microliter; or
(e) a ligation efficiency of between about 85-90% at a ligase concentration of between about 0.1 and 1.0 U/microliter.

8. The composition of claim 1, further comprising a buffer optionally wherein the buffer comprises 1 µM - 1.5 mM ATP.

9. A method for *in situ* analysis of one or more nucleic acids in a biological sample, said method comprising: (a) contacting a biological sample comprising a target nucleic acid with a target padlock probe comprising targeting regions capable of hybridization to the target nucleic acid, wherein the target padlock probe is hybridized to the target nucleic acid; (b) generating a circular template by ligating the target padlock probe with the recombinant ligase composition of any one of claims 1-8; and (c) amplifying the circular template to form an amplification product.

10. The method of claim 9, wherein the padlock probe further comprises one or more target barcode regions.

11. The method of claim 10, wherein the circular template comprises the one or more target barcode regions, optionally wherein the amplification product comprises the one or more target barcode regions or complementary sequences thereof.

12. The method of claim 11, wherein the method further comprises (d) contacting the amplification product with a secondary probe comprising (1) a barcode-binding region capable of hybridization to the target barcode region or a complementary sequence thereof directly or indirectly, and (2) a detection barcode region, wherein the secondary probe is hybridized to the amplification product.

13. The method of claim 12, further comprising (e) contacting the secondary probe with a detection probe comprising (i) a barcode-binding region capable of hybridization to the detection barcode region, and (2) a detectable label, wherein the detection probe is hybridized to the secondary probe.

14. The method of claim 13, further comprising (f) detecting the detectable label, thereby providing a detectable signal for analysis of the target nucleic acid in the sample.
